# EUROPEAN PATENT APPLICATION

(11) **EP 4 596 578 A1**
(43) Date of publication of application: **06.08.2025**
(21) Application number: 23869303.0
(22) Date of filing: 21.09.2023
(51) Int. Cl.: C07K 16/04, C12N 15/06, C12N 15/13, G01N 33/04

(54) **MONOCLONAL ANTIBODY, AND METHOD FOR DETECTING A1 BETA-CASEIN**

(30) Priority: 30.09.2022 JP 2022158177
(71) Applicant: Sowakai Social Medical Corporation, Kurashiki-shi, Okayama 710-0051 (JP); Tokyo University of Agriculture, Tokyo 156-8502 (JP)
(72) Inventor: MATSUYAMA, Makoto, Okayama-shi, Okayama 701-0202 (JP); KOBAYASHI, Tomoe, Okayama-shi, Okayama 701-0202 (JP); KURAMOTO, Takashi, Tokyo 156-8502 (JP)
(74) Representative: Müller-Boré & Partner Patentanwälte PartG mbB
(86) International application number: PCT/JP2023/034219
(87) International publication number: WO 2024/070874

(57) **Abstract**

The objective of the present invention is to provide monoclonal antibodies binding to A1 beta-casein or both of A1 beta-casein and A2 beta-casein, and a method for detecting A1 beta-casein by using the monoclonal antibodies. The monoclonal antibody according to the present invention is characterized in having the specific amino acid sequences of antigen complementarity determining regions.

## Description

### TECHNICAL FIELD

The present invention relates to monoclonal antibodies binding to A1 beta-casein or both of A1 beta-casein and A2 beta-casein, and a method for detecting A1 beta-casein by using the monoclonal antibodies.

### BACKGROUND ART

Milk contains about 3.3% of a protein, and about 80% of the protein contained in milk is casein. Casein is a phosphoprotein formed by binding a phosphate to the side hydroxy group of a serine residue. Casein is classified into alpha-type, beta-type and kappa-type, and beta-casein is further classified into A1-type and A2-type. It has been known that A1 beta-casein among beta-casein causes evolution of gas, a feeling of fullness, diarrhea and gastric irritation. Thus, the market for A2 milk, of which harm caused by A1 beta-casein is reduced, has been expanding in recent years in Europe and the United States, though A2 milk is not yet well-recognized in Japan. It is therefore required to measure the content amount of A1 beta-casein in milk.

The difference between A1 beta-casein and A2 beta-casein is merely whether the amino acid residue at the 67^{th} position is histidine or proline. The ratio of A1 beta-casein and A2 beta-casein in milk is dependent on the difference beta-casein gene of a cow. In other words, a cow is classified into an individual producing A1 beta-casein only, an individual producing A2 beta-casein only, and an individual producing both of A1 beta-casein and A2 beta-casein.

Therefore, the ratio of A1 beta-casein in milk may be evaluated by examining a beta-casein gene of a cow with PCR method. PCR method is accurate, but PCR method can examine a cow only one by one and cannot examine a milk product produced from milk derived from a plurality of cows. In addition, the test by PCR method takes time.

A method for determining an amount of A1 beta-casein or A2 beta-casein in a composition by liquid chromatography-mass spectrometry method (LC-MS method) is described in Patent Document 1. A milk product produced from a plurality of cows can be tested by LC-MS method. But LC-MS method requires a specialized and expensive equipment and takes a lot of trouble and time for preliminarily treating a sample or the like.

ELISA method has been known as a method for determining a quantity of a target substance. A specific antibody against a target substance is used and enzyme reactions are combined in ELISA method. Not only a sample derived from an individual cow but also a milk product can be tested by ELISA method. In addition, ELISA method is easy and does not take a lot of time. An ELISA kit for determining an amount of A1 beta-casein is commercially available (Non-patent Documents 1 and 2).

### PRIOR ART DOCUMENT

### PATENT DOCUMENT

Patent document 1: JP 2022-515563 A

### NON-PATENT DOCUMENT

Non-patent document 1: biosensis (registered trademark) Bovine A1 Beta Casein ELISA Kit, Catalog No: BEK-2364-1P
Non-patent document 2: biosensis (registered trademark) Bovine A1 β-Casein ELISA Kit, Catalogue Number: BEK-2243-1P/2P/5P/10P

### DISCLOSURE OF THE INVENTION

### PROBLEMS TO BE SOLVED BY THE INVENTION

An ELISA kit for determining an amount of A1 beta-casein in milk is commercially available as described above. But A1 beta-casein in a milk sample containing A2 beta-casein, for example, a milk sample containing 1 part or more by mass of A2 beta-casein to A1 beta-casein, cannot be accurately measured by this kit, since A2 beta-casein inhibits the accurate measurement.

The objective of the present invention is to provide monoclonal antibodies binding to A1 beta-casein or both of A1 beta-casein and A2 beta-casein, and a method for detecting A1 beta-casein by using the monoclonal antibodies.

### MEANS FOR SOLVING THE PROBLEMS

The inventors of the present invention repeated intensive studies in order to solve the above-described problems. As a result, the inventors completed the present invention by developing monoclonal antibodies specifically binding to A1 beta-casein or both of A1 beta-casein and A2 beta-casein.

The present invention is hereinafter described.
[1] A monoclonal antibody, comprising:
   a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 303, and
   a light chain variable region comprising a light chain complementarity determining region 1 comprising an amino acid sequence of SEQ ID NO: 304, a light chain complementarity determining region 2 comprising an amino acid sequence of SEQ ID NO: 305, and a light chain complementarity determining region 3 comprising an amino acid sequence of SEQ ID NO: 306,
   wherein the monoclonal antibody binds to A1 beta-casein.
[2] A monoclonal antibody, comprising:
   a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 307, and
   a light chain variable region comprising a light chain complementarity determining region 1 comprising an amino acid sequence of SEQ ID NO: 308, a light chain complementarity determining region 2 comprising an amino acid sequence of SEQ ID NO: 309, and a light chain complementarity determining region 3 comprising an amino acid sequence of SEQ ID NO: 310,
   wherein the monoclonal antibody binds to A1 beta-casein.
[3] A monoclonal antibody, comprising:
   a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 311, and
   a light chain variable region comprising a light chain complementarity determining region 1 comprising an amino acid sequence of SEQ ID NO: 312, a light chain complementarity determining region 2 comprising an amino acid sequence of SEQ ID NO: 313, and a light chain complementarity determining region 3 comprising an amino acid sequence of SEQ ID NO: 314,
   wherein the monoclonal antibody binds to A1 beta-casein and A2 beta-casein.
[4] The monoclonal antibody according to any one of the above [1] to [3], further comprising an Fc region.
[5] A hybridoma comprising a nucleic acid coding the heavy chain variable region and the light chain variable region of the monoclonal antibody according to any one of the above [1] to [3].
[6] A method for detecting A1 beta-casein in a milk sample, the method comprising the step of mixing the milk sample and the monoclonal antibody according to the above [1] or [2].
[7] The method according to the above [6], further comprising the steps of:
   selecting a detector antibody from the monoclonal antibody according to the above [1] or [2] and selecting a capture antibody from the monoclonal antibody according to any one of the above [1] to [3], wherein an epitope of the capture antibody is different from an epitope of the detector antibody,
   immobilizing the capture antibody on a plate, and
   mixing the detector antibody with a mixture of the capture antibody and the milk sample.

### EFFECT OF THE INVENTION

The monoclonal antibodies according to the present invention can specifically bind to A1 beta-casein or both of A1 beta-casein and A2 beta-casein. Thus, A1 beta-casein can be easily detected and an amount thereof can be easily determined in a milk sample by ELISA method with using the monoclonal antibodies according to the present invention. The present invention is therefore very excellent industrially as a technology to easily evaluate milk on the basis of A1 beta-casein, which has attracted attention in recent years.

### BRIEF DESCRIPTION OF THE DRAWINGS

Figure 1 is a graph to demonstrate the result of a quantitative assessment of A1 beta-casein in a milk sample by sandwich ELISA method with using the anti-A1 beta-casein antibody and anti-A1/A2 beta-casein antibody according to the present invention.
Figure 2 is a graph to demonstrate the result of a quantitative assessment of A1 beta-casein in a milk sample by sandwich ELISA method with using the anti-A1 beta-casein antibodies according to the present invention.
Figure 3 is a graph to demonstrate the result of a quantitative assessment of A1 beta-casein in a milk sample by sandwich ELISA method with using the anti-A1 beta-casein antibodies according to the present invention.
Figure 4 is a graph to demonstrate the result of a quantitative assessment of A1 beta-casein in a milk sample by sandwich ELISA method with using the anti-A1 beta-casein antibody and anti-A1/A2 beta-casein antibody according to the present invention.
Figure 5 is a graph to demonstrate the result of a quantitative assessment of A1 beta-casein in a milk sample by sandwich ELISA method with using the anti-A1 beta-casein antibody and anti-A1/A2 beta-casein antibody according to the present invention.

### MODE FOR CARRYING OUT THE INVENTION

Hereinafter, the anti-A1 beta-casein monoclonal antibody prepared from a rat cell by the inventors of the present invention is referred to as "monoclonal antibody (I)", the anti-A1 beta-casein monoclonal antibody prepared from a mouse cell is referred to as "monoclonal antibody (II)", and the anti-A1/A2 beta-casein monoclonal antibody prepared from a rat cell is referred to as "monoclonal antibody (III)" in some cases. The anti-A1/A2 beta-casein monoclonal antibody recognizes both of A1 beta-casein and A2 beta-casein as antigens. The "/" represents "or", and "deletion" means that the amino acid residue may not be present in the definition of the following sequences.

### (1) Monoclonal antibody (I)

The monoclonal antibody (I) comprises a heavy chain variable region comprising a heavy chain complementarity determining region 1 (VH CDR1) comprising an amino acid sequence of SEQ ID NO: 301, a heavy chain complementarity determining region 2 (VH CDR2) comprising an amino acid sequence of SEQ ID NO: 302, and a heavy chain complementarity determining region 3 (VH CDR3) comprising an amino acid sequence of SEQ ID NO: 303; and a light chain variable region comprising a light chain complementarity determining region 1 (VL CDR1) comprising an amino acid sequence of SEQ ID NO: 304, a light chain complementarity determining region 2 (VL CDR2) comprising an amino acid sequence of SEQ ID NO: 305, and a light chain complementarity determining region 3 (VL CDR3) comprising an amino acid sequence of SEQ ID NO: 306; and binds to A1 beta-casein.

SEQ ID NO: 301: Xaa¹-Xaa²-Xaa³-Xaa⁴-Xaa⁵-Xaa⁶-Xaa⁷-Xaa⁸, wherein Xaa¹ = Gly/Ala, Xaa² = Phe/Tyr/Trp/Val/Leu/Ile/Thr/Ser, Xaa³ = Thr/Ser/Val/Leu/Ile/Pro/Asp/Glu, Xaa⁴ = Phe/Tyr/Trp/Val/Leu/Ile, Xaa⁵ = Ser/Thr/Arg/Lys/His/Asn/Gln/Asp/Glu, Xaa⁶ = Asn/Gln/Ser/Thr/Asp/Glu/Arg/Lys/His, Xaa⁷ = Tyr/Phe/Trp/Thr/Ser/Lys/Arg/His/Asn/Gln, Xaa⁸ = Asp/Glu/Gly/Ala/Thr/Ser/Phe/Tyr/Trp

An example of SEQ ID NO: 301 includes the following SEQ ID NO: 501. 
SEQ ID NO: 501: Xaa701-Xaa702-Xaa703-Xaa704-Xaa705-Xaa706-Xaa707-
Xaa708,
 wherein Xaa⁷⁰¹ = Gly/Ala, Xaa⁷⁰² = Phe/Tyr/Trp, Xaa⁷⁰³ = Thr/Ser, Xaa⁷⁰⁴ = Phe/Tyr/Trp, Xaa⁷⁰⁵ = Ser/Thr/Arg/Lys/His, Xaa⁷⁰⁶ = Asn/Gln/Ser/Thr, Xaa⁷⁰⁷ = Tyr/Phe/Trp, Xaa⁷⁰⁸ = Asp/Glu/Gly/Ala/Thr/Ser

SEQ ID NO: 301 is preferably the following SEQ ID NOs: 336 to 344. 
SEQ ID NO: 336: Xaa^299-Xaa^300-Xaa^301-Xaa^302-Xaa^303-Xaa^304-Xaa^305-
Xaa^306,
 wherein Xaa²⁹⁹ = Gly/Ala, Xaa³⁰⁰ = Tyr/Phe/Trp, Xaa³⁰¹ = Thr/Ser, Xaa³⁰² = Tyr/Phe/Trp, Xaa³⁰³ = Thr/Ser, Xaa³⁰⁴ = Asn/Gln, Xaa³⁰⁵ = Tyr/Phe/Trp, Xaa³⁰⁶ = Asp/Glu 
SEQ ID NO: 337: Xaa307-Xaa308-Xaa309-Xaa310-Xaa311-Xaa312-Xaa313-
Xaa314,
 wherein Xaa³⁰⁷ = Gly/Ala, Xaa³⁰⁸ = Tyr/Phe/Trp, Xaa³⁰⁹ = Thr/Ser, Xaa³¹⁰ = Tyr/Phe/Trp, Xaa³¹¹ = Thr/Ser, Xaa³¹² = Asn/Gln, Xaa³¹³ = Tyr/Phe/Trp, Xaa³¹⁴ = Gly/Ala 
SEQ ID NO: 338: Xaa^315-Xaa^316-Xaa^317-Xaa^318-Xaa^319-Xaa^320-Xaa^321-
Xaa^322,
 wherein Xaa³¹⁵ = Gly/Ala, Xaa³¹⁶ = Tyr/Phe/Trp, Xaa³¹⁷ = Thr/Ser, Xaa³¹⁸ = Tyr/Phe/Trp, Xaa³¹⁹ = Thr/Ser, Xaa³²⁰ = Thr/Ser, Xaa³²¹ = Tyr/Phe/Trp, Xaa³²² = Gly/Ala 
SEQ ID NO: 339: Xaa^323-Xaa^324-Xaa^325-Xaa^326-Xaa^327-Xaa^328-Xaa^329-
Xaa^330,
 wherein Xaa³²³ = Gly/Ala, Xaa³²⁴ = Tyr/Phe/Trp, Xaa³²⁵ = Thr/Ser, Xaa³²⁶ = Tyr/Phe/Trp, Xaa³²⁷ = Lys/Arg/His, Xaa³²⁸ = Asn/Gln, Xaa³²⁹ = Tyr/Phe/Trp, Xaa³³⁰ = Asp/Glu 
SEQ ID NO: 340: Xaa^331-Xaa^332-Xaa^333-Xaa^334-Xaa^335-Xaa^336-Xaa^337-
Xaa^338,
 wherein Xaa³³¹ = Gly/Ala, Xaa³³² = Tyr/Phe/Trp, Xaa³³³ = Thr/Ser, Xaa³³⁴ = Tyr/Phe/Trp, Xaa³³⁵ = Thr/Ser, Xaa³³⁶ = Thr/Ser, Xaa³³⁷ = Tyr/Phe/Trp, Xaa³³⁸ = Thr/Ser 
SEQ ID NO: 341: Xaa^339-Xaa^340-Xaa^341-Xaa^342-Xaa^343-Xaa^344-Xaa^345-
Xaa^346,
 wherein Xaa³³⁹ = Gly/Ala, Xaa³⁴⁰ = Tyr/Phe/Trp, Xaa³⁴¹ = Val/Leu/Ile, Xaa³⁴² = Tyr/Phe/Trp, Xaa³⁴³ = Arg/Lys/His, Xaa³⁴⁴ = Asn/Gln, Xaa³⁴⁵ = Tyr/Phe/Trp, Xaa³⁴⁶ = Gly/Ala 
SEQ ID NO: 342: Xaa^347-Xaa^348-Xaa^349-Xaa^350-Xaa^351-Xaa^352-Xaa^353-
Xaa^354,
 wherein Xaa³⁴⁷ = Gly/Ala, Xaa³⁴⁸ = Tyr/Phe/Trp, Xaa³⁴⁹ = Thr/Ser, Xaa³⁵⁰ = Tyr/Phe/Trp, Xaa³⁵¹ = Thr/Ser, Xaa³⁵² = Asn/Gln, Xaa³⁵³ = Tyr/Phe/Trp, Xaa³⁵⁴ = Tyr/Phe/Trp 
SEQ ID NO: 343: Xaa^355-Xaa^356-Xaa^357-Xaa^358-Xaa^359-Xaa^360-Xaa^361-
Xaa^362,
 wherein Xaa³⁵⁵ = Gly/Ala, Xaa³⁵⁶ = Tyr/Phe/Trp, Xaa³⁵⁷ = Thr/Ser, Xaa³⁵⁸ = Tyr/Phe/Trp, Xaa³⁵⁹ = Thr/Ser, Xaa³⁶⁰ = Asn/Gln, Xaa³⁶¹ = Tyr/Phe/Trp, Xaa³⁶² = Gly/Ala 
SEQ ID NO: 344: Xaa363-Xaa364-Xaa365-Xaa366-Xaa367-Xaa368-Xaa369-
Xaa370,
 wherein Xaa³⁶³ = Gly/Ala, Xaa³⁶⁴ = Tyr/Phe/Trp, Xaa³⁶⁵ = Thr/Ser, Xaa³⁶⁶ = Tyr/Phe/Trp, Xaa³⁶⁷ = Asp/Glu, Xaa³⁶⁸ = Asp/Glu, Xaa³⁶⁹ = Tyr/Phe/Trp, Xaa³⁷⁰ = Gly/Ala

SEQ ID NO: 302: Xaa⁹-Xaa¹⁰-Xaa¹¹-Xaa¹²-Xaa¹³-Xaa¹⁴-Xaa¹⁵-Xaa¹⁶, wherein Xaa⁹ = Ile/Val/Leu/Phe/Tyr/Trp, Xaa¹⁰ = Thr/Ser/Asn/Gln/Lys/Arg/His/Gly/Ala/Tyr/Phe/Trp, Xaa¹¹ = Thr/Ser/Pro/Tyr/Phe/Trp/Gly/Ala/Asn/Gln/Val/Leu/Ile/Asp/Glu, Xaa¹² = Ser/Thr/Asp/Glu/Lys/Arg/His/Phe/Tyr/Trp/Gly/Ala/Asn/Gln, Xaa¹³ = Gly/Ala/Ser/Thr/Asn/Gln/Lys/Arg/His/Asp/Glu, Xaa¹⁴ = Gly/Ala/Ser/Thr/Val/Leu/Ile/Lys/Arg/His, Xaa¹⁵ = Ser/Thr/Tyr/Phe/Trp/Lys/Arg/His/Gly/Ala/Asn/Gln/Val/Leu/Ile/As p/Glu/Cys/Met, Xaa¹⁶ = Asp/Glu/Gly/Ala/Thr/Ser/Pro/Val/Leu/Ile

An example of SEQ ID NO: 302 includes the following SEQ ID NO: 502. 
SEQ ID NO: 502: Xaa⁷⁰⁹-Xaa⁷¹⁰-Xaa⁷¹¹-Xaa⁷¹²-Xaa⁷¹³-Xaa⁷¹⁴-Xaa⁷¹⁵-
Xaa⁷¹⁶,
 wherein Xaa⁷⁰⁹ = Ile/Val/Leu/Phe/Tyr/Trp, Xaa⁷¹⁰ = Thr/Ser/Asn/Gln, Xaa⁷¹¹ = Thr/Ser/Pro/Tyr/Phe/Trp, Xaa⁷¹² = Ser/Thr/Asp/Glu, Xaa⁷¹³ = Gly/Ala/Ser/Thr, Xaa⁷¹⁴ = Gly/Ala/Ser/Thr, Xaa⁷¹⁵ = Ser/Thr/Tyr/Phe/Trp, Xaa⁷¹⁶ = Asp/Glu/Gly/Ala/Thr/Ser

SEQ ID NO: 302 is preferably the following SEQ ID NOs: 345 to 353. 
SEQ ID NO: 345: Xaa^371-Xaa^372-Xaa^373-Xaa^374-Xaa^375-Xaa^376-Xaa^377-
Xaa^378,
 wherein Xaa³⁷¹ = Val/Leu/Ile, Xaa³⁷² = Thr/Ser, Xaa³⁷³ = Thr/Ser, Xaa³⁷⁴ = Thr/Ser, Xaa³⁷⁵ = Gly/Ala, Xaa³⁷⁶ = Gly/Ala, Xaa³⁷⁷ = Thr/Ser, Xaa³⁷⁸ = Thr/Ser 
SEQ ID NO: 346: Xaa³⁷⁹-Xaa³⁸⁰-Xaa³⁸¹-Xaa³⁸²-Xaa³⁸³-Xaa³⁸⁴-Xaa³⁸⁵-
Xaa³⁸⁶,
 wherein Xaa³⁷⁹ = Val/Leu/Ile, Xaa³⁸⁰ = Asn/Gln, Xaa³⁸¹ = Tyr/Phe/Trp, Xaa³⁸² = Asp/Glu, Xaa³⁸³ = Gly/Ala, Xaa³⁸⁴ = Thr/Ser, Xaa³⁸⁵ = Thr/Ser, Xaa³⁸⁶ = Thr/Ser 
SEQ ID NO: 347: Xaa³⁸⁷-Xaa³⁸⁸-Xaa³⁸⁹-Xaa³⁹⁰-Xaa³⁹¹-Xaa³⁹²-Xaa³⁹³-
Xaa³⁹⁴,
 wherein Xaa³⁸⁷ = Val/Leu/Ile, Xaa³⁸⁸ = Thr/Ser, Xaa³⁸⁹ = Pro, Xaa³⁹⁰ = Thr/Ser, Xaa³⁹¹ = Gly/Ala, Xaa³⁹² = Gly/Ala, Xaa³⁹³ = Thr/Ser, Xaa³⁹⁴ = Val/Leu/Ile 
SEQ ID NO: 348: Xaa395-Xaa396-Xaa397-Xaa398-Xaa399-Xaa400-Xaa401-
Xaa402,
 wherein Xaa³⁹⁵ = Val/Leu/Ile, Xaa³⁹⁶ = Asn/Gln, Xaa³⁹⁷ = Pro, Xaa³⁹⁸ = Thr/Ser, Xaa³⁹⁹ = Thr/Ser, Xaa⁴⁰⁰ = Gly/Ala, Xaa⁴⁰¹ = Tyr/Phe/Trp, Xaa⁴⁰² = Val/Leu/Ile 
SEQ ID NO: 349: Xaa⁴⁰³-Xaa⁴⁰⁴-Xaa⁴⁰⁵-Xaa⁴⁰⁶-Xaa⁴⁰⁷-Xaa⁴⁰⁸-Xaa⁴⁰⁹-
Xaa⁴¹⁰,
 wherein Xaa⁴⁰³ = Val/Leu/Ile, Xaa⁴⁰⁴ = Asn/Gln, Xaa⁴⁰⁵ = Pro, Xaa⁴⁰⁶ = Thr/Ser, Xaa⁴⁰⁷ = Thr/Ser, Xaa⁴⁰⁸ = Gly/Ala, Xaa⁴⁰⁹ = Tyr/Phe/Trp, Xaa⁴¹⁰ = Thr/Ser 
SEQ ID NO: 350: Xaa⁴¹¹-Xaa⁴¹²-Xaa⁴¹³-Xaa⁴¹⁴-Xaa⁴¹⁵-Xaa⁴¹⁶-Xaa⁴¹⁷-
Xaa⁴¹⁸,
 wherein Xaa⁴¹¹ = Tyr/Phe/Trp, Xaa⁴¹² = Asn/Gln, Xaa⁴¹³ = Pro, Xaa⁴¹⁴ = Thr/Ser, Xaa⁴¹⁵ = Thr/Ser, Xaa⁴¹⁶ = Gly/Ala, Xaa⁴¹⁷ = Tyr/Phe/Trp, Xaa⁴¹⁸ = Thr/Ser 
SEQ ID NO: 351: Xaa⁴¹⁹-Xaa⁴²⁰-Xaa⁴²¹-Xaa⁴²²-Xaa⁴²³-Xaa⁴²⁴-Xaa⁴²⁵-
Xaa⁴²⁶,
 wherein Xaa⁴¹⁹ = Val/Leu/Ile, Xaa⁴²⁰ = Asn/Gln, Xaa⁴²¹ = Thr/Ser, Xaa⁴²² = Tyr/Phe/Trp, Xaa⁴²³ = Thr/Ser, Xaa⁴²⁴ = Gly/Ala, Xaa⁴²⁵ = Asp/Glu, Xaa⁴²⁶ = Thr/Ser 
SEQ ID NO: 352: Xaa427-Xaa428-Xaa429-Xaa430-Xaa431-Xaa432-Xaa433-
Xaa434,
 wherein Xaa⁴²⁷ = Val/Leu/Ile, Xaa⁴²⁸ = Thr/Ser, Xaa⁴²⁹ = Asn/Gln, Xaa⁴³⁰ = Gly/Ala, Xaa⁴³¹ = Gly/Ala, Xaa⁴³² = Gly/Ala, Xaa⁴³³ = Thr/Ser, Xaa⁴³⁴ = Thr/Ser 
SEQ ID NO: 353: Xaa435-Xaa436-Xaa437-Xaa438-Xaa439-Xaa440-Xaa441-
Xaa442,
 wherein Xaa⁴³⁵ = Val/Leu/Ile, Xaa⁴³⁶ = Thr/Ser, Xaa⁴³⁷ = Tyr/Phe/Trp, Xaa⁴³⁸ = Gly/Ala, Xaa⁴³⁹ = Gly/Ala, Xaa⁴⁴⁰ = Arg/Lys/His, Xaa⁴⁴¹ = Thr/Ser, Xaa⁴⁴² = Val/Leu/Ile 
SEQ ID NO: 303: Xaa^17-Xaa^18-Xaa^19-Xaa^20-Xaa^21-Xaa^22-Xaa^23-Xaa^24-
Xaa^25-Xaa^26-Xaa^27-Xaa^28-Xaa^29-Xaa^30-Xaa^31-Xaa^32-Xaa^33-Xaa^34-Xaa^35-
Xaa^36-Xaa^37,
 wherein Xaa¹⁷ = Ala/Gly/deletion, Xaa¹⁸ = Arg/Lys/His/Ser/Thr, Xaa¹⁹ = His/Arg/Lys/deletion, Xaa²⁰ = Pro/Gly/Ala/Ser/Thr/Asp/Glu, Xaa²¹ = Tyr/Phe/Trp/Arg/Lys/His/Gly/Ala/deletion, Xaa²² = Asn/Gln/Tyr/Phe/Trp/Asp/Glu/Arg/Lys/His/deletion, Xaa²³ = Asn/Gln/Val/Leu/Ile/deletion, Xaa²⁴ = Tyr/Phe/Trp/Val/Leu/Ile/deletion, Xaa²⁵ = Asp/Glu/Gly/Ala/deletion, Xaa²⁶ = Gly/Ala/deletion, Xaa²⁷ = Ser/Thr/Asn/Gln/deletion, Xaa²⁸ = Tyr/Phe/Trp/deletion, Xaa²⁹ = Tyr/Phe/Trp/Asn/Gln/Ser/Thr/deletion, Xaa³⁰ = Arg/Lys/His/Asp/Glu/deletion, Xaa³¹ = Tyr/Phe/Trp/Gly/Ala/deletion, Xaa³² = Gly/Ala/Tyr/Phe/Trp/Pro/Ser/Thr/deletion, Xaa³³ = Gly/Ala/Tyr/Phe/Trp/deletion, Xaa³⁴ = Asp/Glu/Ser/Thr/Tyr/Phe/Trp/Arg/Lys/His/Val/Leu/Ile/deletion, Xaa³⁵ = Phe/Tyr/Trp/deletion, Xaa³⁶ = Asp/Glu/Gly/Ala, Xaa³⁷ = Phe/Tyr/Trp

An example of SEQ ID NO: 303 includes the following SEQ ID NO: 503. 
SEQ ID NO: 503: Xaa717-Xaa718-Xaa719-Xaa720-Xaa721-Xaa722-Xaa723-
Xaa724-Xaa725-Xaa726-Xaa727-Xaa728-Xaa729-Xaa730-Xaa731-Xaa732-Xaa733-
Xaa734-Xaa735-Xaa736,
 wherein Xaa⁷¹⁷ = Ala/Gly, Xaa⁷¹⁸ = Arg/Lys/His, Xaa⁷¹⁹ = His/Arg/Lys/deletion, Xaa⁷²⁰ = Pro/Gly/Ala/Ser/Thr, Xaa⁷²¹ = Tyr/Phe/Trp, Xaa⁷²² = Asn/Gln/deletion, Xaa⁷²³ = Asn/Gln/deletion, Xaa⁷²⁴ = Tyr/Phe/Trp, Xaa⁷²⁵ = Asp/Glu/deletion, Xaa⁷²⁶ = Gly/Ala, Xaa⁷²⁷ = Ser/Thr/Asn/Gln/deletion, Xaa⁷²⁸ = Tyr/Phe/Trp, Xaa⁷²⁹ = Tyr/Phe/Trp, Xaa⁷³⁰ = Tyr/Phe/Trp, Xaa⁷³¹ = Gly/Ala/deletion, Xaa⁷³² = Gly/Ala/deletion, Xaa⁷³³ = Asp/Glu/Ser/Thr/deletion, Xaa⁷³⁴ = Phe/Tyr/Trp, Xaa⁷³⁵ = Asp/Glu, Xaa⁷³⁶ = Phe/Tyr/Trp

SEQ ID NO: 303 is preferably the following SEQ ID NOs: 315, 316 and 354 to 356. 
SEQ ID NO: 315: Xaa126-Xaa127-Xaa128-Xaa129-Xaa130-Xaa131-Xaa132-
Xaa133-Xaa134-Xaa135-Xaa136-Xaa137-Xaa138-Xaa139,
 wherein Xaa¹²⁶ = Ala/Gly, Xaa¹²⁷ = Arg/Lys/His, Xaa¹²⁸ = Pro/Gly/Ala, Xaa¹²⁹ = Tyr/Phe/Trp, Xaa¹³⁰ = Asn/Gln, Xaa¹³¹ = Asn/Gln, Xaa¹³² = Tyr/Phe/Trp, Xaa¹³³ = Gly/Ala, Xaa¹³⁴ = Tyr/Phe/Trp, Xaa¹³⁵ = Tyr/Phe/Trp, Xaa¹³⁶ = Tyr/Phe/Trp, Xaa¹³⁷ = Phe/Tyr/Trp, Xaa¹³⁸ = Asp/Glu, Xaa¹³⁹ = Phe/Tyr/Trp 
SEQ ID NO: 316: Xaa^140-Xaa^141-Xaa^142-Xaa^143-Xaa^144-Xaa^145-Xaa^146-
Xaa^147-Xaa^148-Xaa^149-Xaa^150-Xaa^151-Xaa^152-Xaa^153-Xaa^154-Xaa^155-Xaa^156-
Xaa^157,
 wherein Xaa¹⁴⁰ = Ala/Gly, Xaa¹⁴¹ = Arg/Lys/His, Xaa¹⁴² = His/Arg/Lys, Xaa¹⁴³ = Pro/Gly/Ala/Ser/Thr, Xaa¹⁴⁴ = Tyr/Phe/Trp, Xaa¹⁴⁵ = Tyr/Phe/Trp, Xaa¹⁴⁶ = Asp/Glu, Xaa¹⁴⁷ = Gly/Ala, Xaa¹⁴⁸ = Ser/Thr/Asn/Gln, Xaa¹⁴⁹ = Tyr/Phe/Trp, Xaa¹⁵⁰ = Tyr/Phe/Trp, Xaa¹⁵¹ = Tyr/Phe/Trp, Xaa¹⁵² = Gly/Ala, Xaa¹⁵³ = Gly/Ala, Xaa¹⁵⁴ = Asp/Glu/Ser/Thr, Xaa¹⁵⁵ = Phe/Tyr/Trp, Xaa¹⁵⁶ = Asp/Glu, Xaa¹⁵⁷ = Phe/Tyr/Trp 
SEQ ID NO: 354: Xaa443-Xaa444-Xaa445-Xaa446-Xaa447-Xaa448-Xaa449-
Xaa450-Xaa451-Xaa452-Xaa453-Xaa454-Xaa455-Xaa456-Xaa457-Xaa458-Xaa459-
 Xaa460, wherein Xaa⁴⁴³ = Gly/Ala, Xaa⁴⁴⁴ = His/Arg/Lys, Xaa⁴⁴⁵ = His/Arg/Lys, Xaa⁴⁴⁶ = Thr/Ser, Xaa⁴⁴⁷ = Tyr/Phe/Trp, Xaa⁴⁴⁸ = Tyr/Phe/Trp, Xaa⁴⁴⁹ = Asp/Glu, Xaa⁴⁵⁰ = Gly/Ala, Xaa⁴⁵¹ = Asn/Gln, Xaa⁴⁵² = Tyr/Phe/Trp, Xaa⁴⁵³ = Tyr/Phe/Trp, Xaa⁴⁵⁴ = Tyr/Phe/Trp, Xaa⁴⁵⁵ = Gly/Ala, Xaa⁴⁵⁶ = Gly/Ala, Xaa⁴⁵⁷ = Ser/Thr, Xaa⁴⁵⁸ = Tyr/Phe/Trp, Xaa⁴⁵⁹ = Asp/Glu, Xaa⁴⁶⁰ = Tyr/Phe/Trp 
SEQ ID NO: 355: Xaa461-Xaa462-Xaa463-Xaa464-Xaa465-Xaa466-Xaa467-
Xaa468-Xaa469-Xaa470-Xaa471-Xaa472-Xaa473-Xaa474-Xaa475-Xaa476-Xaa477-
Xaa478,
 wherein Xaa⁴⁶¹ = Gly/Ala, Xaa⁴⁶² = His/Arg/Lys, Xaa⁴⁶³ = His/Arg/Lys, Xaa⁴⁶⁴ = Gly/Ala, Xaa⁴⁶⁵ = Tyr/Phe/Trp, Xaa⁴⁶⁶ = Tyr/Phe/Trp, Xaa⁴⁶⁷ = Asp/Glu, Xaa⁴⁶⁸ = Gly/Ala, Xaa⁴⁶⁹ = Ser/Thr, Xaa⁴⁷⁰ = Tyr/Phe/Trp, Xaa⁴⁷¹ = Tyr/Phe/Trp, Xaa⁴⁷² = Tyr/Phe/Trp, Xaa⁴⁷³ = Gly/Ala, Xaa⁴⁷⁴ = Gly/Ala, Xaa⁴⁷⁵ = Asp/Glu, Xaa⁴⁷⁶ = Tyr/Phe/Trp, Xaa⁴⁷⁷ = Asp/Glu, Xaa⁴⁷⁸ = Tyr/Phe/Trp 
SEQ ID NO: 356: Xaa^479-Xaa^480-Xaa^481-Xaa^482-Xaa^483-Xaa^484-Xaa^485-
Xaa^486-Xaa^487-Xaa^488-Xaa^489,
 wherein Xaa⁴⁷⁹ = Ser/Thr, Xaa⁴⁸⁰ = His/Arg/Lys, Xaa⁴⁸¹ = Asp/Glu, Xaa⁴⁸² = His/Arg/Lys, Xaa⁴⁸³ = Gly/Ala, Xaa⁴⁸⁴ = His/Arg/Lys, Xaa⁴⁸⁵ = Tyr/Phe/Trp, Xaa⁴⁸⁶ = Val/Leu/Ile, Xaa⁴⁸⁷ = Tyr/Phe/Trp, Xaa⁴⁸⁸ = Asp/Glu, Xaa⁴⁸⁹ = Tyr/Phe/Trp 
SEQ ID NO: 304: Xaa38-Xaa39-Xaa40-Xaa41-Xaa42-Xaa43-Xaa44-Xaa45-
Xaa46-Xaa47-Xaa48,
 wherein Xaa³⁸ = Gln/Asn/Ser/Thr/Lys/Arg/His, Xaa³⁹ = Asn/Gln/Ser/Thr/Arg/Lys/His, Xaa⁴⁰ = Ile/Val/Leu, Xaa⁴¹ = Ser/Thr/Ile/Val/Leu/Asp/Glu/deletion, Xaa⁴² = Lys/Arg/His/Tyr/Phe/Trp/deletion, Xaa⁴³ = Ser/Thr/Tyr/Phe/Trp/deletion, Xaa⁴⁴ = Ser/Thr/Asn/Gln/Asp/Glu/deletion, Xaa⁴⁵ = Gly/Ala/deletion, Xaa⁴⁶ = Tyr/Phe/Trp/Asn/Gln/Lys/Arg/His/deletion, Xaa⁴⁷ = Lys/Arg/His/Ser/Thr/deletion, Xaa⁴⁸ = Asn/Gln/Tyr/Phe/Trp/deletion

An example of SEQ ID NO: 304 includes the following SEQ ID NO: 504.

SEQ ID NO: 504: Xaa⁷³⁷-Xaa⁷³⁸-Xaa⁷³⁹-Xaa⁷⁴⁰-Xaa⁷⁴¹-Xaa⁷⁴²-Xaa⁷⁴³, wherein Xaa⁷³⁷ = Gln/Asn/Ser/Thr, Xaa⁷³⁸ = Asn/Gln/Ser/Thr/Arg/Lys/His, Xaa⁷³⁹ = Ile/Val/Leu, Xaa⁷⁴⁰ = Ser/Thr/deletion, Xaa⁷⁴¹ = Tyr/Phe/Trp, Xaa⁷⁴² = Lys/Arg/His/deletion, Xaa⁷⁴³ = Asn/Gln/deletion

SEQ ID NO: 304 is preferably the following SEQ ID NOs: 317, 318 and 357 to 359.

SEQ ID NO: 317: Xaa¹⁵⁸-Xaa¹⁵⁹-Xaa¹⁶⁰-Xaa¹⁶¹-Xaa¹⁶²-Xaa¹⁶³, wherein Xaa¹⁵⁸ = Gln/Asn, Xaa¹⁵⁹ = Asn/Gln, Xaa¹⁶⁰ = Ile/Val/Leu, Xaa¹⁶¹ = Tyr/Phe/Trp, Xaa¹⁶² = Lys/Arg/His, Xaa¹⁶³ = Asn/Gln

SEQ ID NO: 318: Xaa¹⁶⁴-Xaa¹⁶⁵-Xaa¹⁶⁶-Xaa¹⁶⁷-Xaa¹⁶⁸, wherein Xaa¹⁶⁴ = Ser/Thr, Xaa¹⁶⁵ = Ser/Thr/Arg/Lys/His, Xaa¹⁶⁶ = Ile/Val/Leu, Xaa¹⁶⁷ = Ser/Thr, Xaa¹⁶⁸ = Tyr/Phe/Trp

SEQ ID NO: 357: Xaa⁴⁹⁰-Xaa⁴⁹¹-Xaa⁴⁹²-Xaa⁴⁹³-Xaa⁴⁹⁴, wherein Xaa⁴⁹⁰ = Ser/Thr, Xaa⁴⁹¹ = His/Arg/Lys, Xaa⁴⁹² = Ile/Val/Leu, Xaa⁴⁹³ = Ser/Thr, Xaa⁴⁹⁴ = Tyr/Phe/Trp

SEQ ID NO: 358: Xaa⁴⁹⁵-Xaa⁴⁹⁶-Xaa⁴⁹⁷-Xaa⁴⁹⁸-Xaa⁴⁹⁹, wherein Xaa⁴⁹⁵ = Ser/Thr, Xaa⁴⁹⁶ = Ser/Thr, Xaa⁴⁹⁷ = Ile/Val/Leu, Xaa⁴⁹⁸ = Ser/Thr, Xaa⁴⁹⁹ = Tyr/Phe/Trp

SEQ ID NO: 359: Xaa⁵⁰⁰-Xaa⁵⁰¹-Xaa⁵⁰²-Xaa⁵⁰³-Xaa⁵⁰⁴-Xaa⁵⁰⁵, wherein Xaa⁵⁰⁰ = Asn/Gln, Xaa⁵⁰¹ = Asn/Gln, Xaa⁵⁰² = Ile/Val/Leu, Xaa⁵⁰³ = Asn/Gln, Xaa⁵⁰⁴ = Asn/Gln, Xaa⁵⁰⁵ = Tyr/Phe/Trp

SEQ ID NO: 305: Xaa⁴⁹-Xaa⁵⁰-Xaa⁵¹, wherein Xaa⁴⁹ = Asn/Gln/Asp/Glu/lle/Val/Leu/Thr/Ser/Tyr/Phe/Trp, Xaa⁵⁰ = Ala/Gly/Thr/Ser/Ile/Val/Leu/Pro/Cys/Met, Xaa⁵¹ = Asn/Gln/Ser/Thr

An example of SEQ ID NO: 305 includes the following SEQ ID NO: 505.

SEQ ID NO: 505: Xaa⁷⁴⁴-Xaa⁷⁴⁵-Xaa⁷⁴⁶, wherein Xaa⁷⁴⁴ = Asn/Gln/Asp/Glu, Xaa⁷⁴⁵ = Ala/Gly/Thr/Ser, Xaa⁷⁴⁶ = Asn/Gln/Ser/Thr

SEQ ID NO: 305 is preferably the following SEQ ID NOs: 319, 320 and 360.

SEQ ID NO: 319: Xaa¹⁶⁹-Xaa¹⁷⁰-Xaa¹⁷¹, wherein Xaa¹⁶⁹ = Gln/Asn, Xaa¹⁷⁰ = Ala/Gly, Xaa¹⁷¹ = Gln/Asn

SEQ ID NO: 320: Xaa¹⁷²-Xaa¹⁷³-Xaa¹⁷⁴, wherein Xaa¹⁷² = Asp/Glu, Xaa¹⁷³ = Thr/Ser, Xaa¹⁷⁴ = Ser/Thr

SEQ ID NO: 360: Xaa⁵⁰⁶-Xaa⁵⁰⁷-Xaa⁵⁰⁸, wherein Xaa⁵⁰⁶ = Tyr/Phe/Trp, Xaa⁵⁰⁷ = Aly/Gly, Xaa⁵⁰⁸ = Ser/Thr 
SEQ ID NO: 306: Xaa⁵²-Xaa⁵³-Xaa⁵⁴-Xaa⁵⁵-Xaa⁵⁶-Xaa⁵⁷-Xaa⁵⁸-Xaa⁵⁹-
Xaa⁶⁰-Xaa⁶¹,
 wherein Xaa⁵² = Gln/Asn/Ile/Val/Leu/Tyr/Phe/Trp/deletion, Xaa⁵³ = Gln/Asn/Lys/Arg/His, Xaa⁵⁴ = Tyr/Phe/Trp/Ser/Thr/Gly/Ala/Lys/Arg/His, Xaa⁵⁵ = Tyr/Phe/Trp/Ser/Thr/Gln/Asn/Leu/Val/Ile/Lys/Arg/His/deletion, Xaa⁵⁶ = Ser/Thr/Lys/Arg/His/Asp/Glu/deletion, Xaa⁵⁷ = Gln/Asn/Ser/Thr/Gly/Ala/Tyr/Phe/Trp/lle/Val/Leu/deletion, Xaa⁵⁸ = Thr/Ser/Pro/deletion, Xaa⁵⁹ = Gly/Ala/Pro/Gln/Asn/deletion, Xaa⁶⁰ = Leu/Val/Ile/Tyr/Phe/Trp/deletion, Xaa⁶¹ = Thr/Ser

An example of SEQ ID NO: 306 includes the following SEQ ID NO: 506. 
SEQ ID NO: 506: Xaa^747-Xaa^748-Xaa^749-Xaa^750-Xaa^751-Xaa^752-Xaa^753-
Xaa^754-Xaa^755-Xaa^756,
 wherein Xaa⁷⁴⁷ = Gln/Asn, Xaa⁷⁴⁸ = Gln/Asn, Xaa⁷⁴⁹ = Tyr/Phe/Trp, Xaa⁷⁵⁰ = Tyr/Phe/Trp/deletion, Xaa⁷⁵¹ = Ser/Thr, Xaa⁷⁵² = Gln/Asn/Ser/Thr/deletion, Xaa⁷⁵³ = Thr/Ser/deletion, Xaa⁷⁵⁴ = Gly/Ala/Pro, Xaa⁷⁵⁵ = Leu/Val/Ile/Tyr/Phe/Trp, Xaa⁷⁵⁶ = Thr/Ser

SEQ ID NO: 306 is preferably the following SEQ ID NOs: 321, 322 and 361 to 363. 
SEQ ID NO: 321: Xaa^175-Xaa^176-Xaa^177-Xaa^178-Xaa^179-Xaa^180-Xaa^181-
Xaa^182,
 wherein Xaa¹⁷⁵ = Gln/Asn, Xaa¹⁷⁶ = Gln/Asn, Xaa¹⁷⁷ = Tyr/Phe/Trp, Xaa¹⁷⁸ = Tyr/Phe/Trp, Xaa¹⁷⁹ = Ser/Thr, Xaa¹⁸⁰ = Gly/Ala/Pro, Xaa¹⁸¹ = Leu/Val/Ile, Xaa¹⁸² = Thr/Ser 
SEQ ID NO: 322: Xaa183-Xaa184-Xaa185-Xaa186-Xaa187-Xaa188-Xaa189-
Xaa190-Xaa191,
 wherein Xaa¹⁸³ = Gln/Asn, Xaa¹⁸⁴ = Gln/Asn, Xaa¹⁸⁵ = Tyr/Phe/Trp, Xaa¹⁸⁶ = Ser/Thr, Xaa¹⁸⁷ = Gln/Asn/Ser/Thr, Xaa¹⁸⁸ = Thr/Ser, Xaa¹⁸⁹ = Gly/Ala/Pro, Xaa¹⁹⁰ = Tyr/Phe/Trp, Xaa¹⁹¹ = Thr/Ser 
SEQ ID NO: 361: Xaa^509-Xaa^510-Xaa^511-Xaa^512-Xaa^513-Xaa^514-Xaa^515-
Xaa^516-Xaa^517,
 wherein Xaa⁵⁰⁹ = Gln/Asn, Xaa⁵¹⁰ = Gln/Asn, Xaa⁵¹¹ = Tyr/Phe/Trp, Xaa⁵¹² = Ser/Thr, Xaa⁵¹³ = Ser/Thr, Xaa⁵¹⁴ = Ser/Thr, Xaa⁵¹⁵ = Pro, Xaa⁵¹⁶ = Tyr/Phe/Trp, Xaa⁵¹⁷ = Ser/Thr 
SEQ ID NO: 362: Xaa⁵¹⁸-Xaa⁵¹⁹-Xaa⁵²⁰-Xaa⁵²¹-Xaa⁵²²-Xaa⁵²³-Xaa⁵²⁴-
Xaa⁵²⁵-Xaa⁵²⁶,
 wherein Xaa⁵¹⁸ = Gln/Asn, Xaa⁵¹⁹ = Gln/Asn, Xaa⁵²⁰ = Tyr/Phe/Trp, Xaa⁵²¹ = Ser/Thr, Xaa⁵²² = Gln/Asn, Xaa⁵²³ = Ser/Thr, Xaa⁵²⁴ = Pro, Xaa⁵²⁵ = Tyr/Phe/Trp, Xaa⁵²⁶ = Ser/Thr 
SEQ ID NO: 363: Xaa527-Xaa528-Xaa529-Xaa530-Xaa531-Xaa532-Xaa533-
Xaa534,
 wherein Xaa⁵²⁷ = Gln/Asn, Xaa⁵²⁸ = Gln/Asn, Xaa⁵²⁹ = Tyr/Phe/Trp, Xaa⁵³⁰ = Gln/Asn, Xaa⁵³¹ = Ser/Thr, Xaa⁵³² = Tyr/Phe/Trp, Xaa⁵³³ = Leu/Val/Ile, Xaa⁵³⁴ = Ser/Thr

### (2) Monoclonal antibody (II)

The monoclonal antibody (II) comprises a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 307; and a light chain variable region comprising a light chain complementarity determining region 1 comprising an amino acid sequence of SEQ ID NO: 308, a light chain complementarity determining region 2 comprising an amino acid sequence of SEQ ID NO: 309, and a light chain complementarity determining region 3 comprising an amino acid sequence of SEQ ID NO: 310; wherein the monoclonal antibody binds to A1 beta-casein.

SEQ ID NO: 307: Xaa⁶²-Xaa⁶³-Xaa⁶⁴-Xaa⁶⁵-Xaa⁶⁶-Xaa⁶⁷-Xaa⁶⁸-Xaa⁶⁹-Xaa⁷⁰-Xaa⁷¹-Xaa⁷²-Xaa⁷³-Xaa⁷⁴-Xaa⁷⁵-Xaa⁷⁶-Xaa⁷⁷-Xaa⁷⁸, wherein Xaa⁶² = Ala/Gly/Ser/Thr/Val/Leu/Ile, Xaa⁶³ = Val/Leu/Ile/Gly/Ala/Lys/Arg/His/deletion, Xaa⁶⁴ = Tyr/Phe/Trp/Pro/Asp/Glu/Lys/Arg/His/deletion, Xaa⁶⁵ = Asp/Glu/Ala/Gly/Ser/Thr/Lys/Arg/His/Pro/deletion, Xaa⁶⁶ = Gly/Ala/Lys/Arg/His/Asp/Glu/Pro/Val/Leu/Ile/Tyr/Phe/Trp/deleti on, Xaa⁶⁷ = Tyr/Phe/Trp/Lys/Arg/His/Ser/Thr/deletion, Xaa⁶⁸ = Tyr/Phe/Trp/Asn/Gln/Pro/Gly/Ala/Lys/Arg/His/deletion, Xaa⁶⁹ = Gly/Ala/Tyr/Phe/Trp/Asp/Glu/Ser/Thr/deletion, Xaa⁷⁰ = Asn/Gln/Ala/Gly/Ser/Thr/Phe/Tyr/Trp/Asp/Glu/Lys/Arg/His/deleti on, Xaa⁷¹ = Phe/Tyr/Trp/Ser/Thr/Val/Leu/Ile/Asp/Glu/Ala/Gly/deletion, Xaa⁷² = Tyr/Phe/Trp/Asp/Glu/Lys/Arg/His/Ser/Thr/Gly/Ala/Pro, Xaa⁷³ = Val/Leu/Ile/Asp/Glu/Ser/Thr/Cys/Met/Lys/Arg/His/Gly/Ala/deleti on, Xaa⁷⁴ = Tyr/Phe/Trp/Gly/Ala/Ser/Thr/deletion, Xaa⁷⁵ = Pro/Val/Leu/Ile/Gly/Ala/Phe/Tyr/Trp/deletion, Xaa⁷⁶ = Phe/Tyr/Trp/Met/Cys/Val/Leu/Ile/Lys/Arg/His/deletion, Xaa⁷⁷ = Asp/Glu/Gly/Ala/Pro/Val/Leu/Ile, Xaa⁷⁸ = Tyr/Phe/Trp

An example of SEQ ID NO: 307 includes the following SEQ ID NO: 507.

SEQ ID NO: 507: Xaa⁷⁵⁷-Xaa⁷⁵⁸-Xaa⁷⁵⁹-Xaa⁷⁶⁰-Xaa⁷⁶¹-Xaa⁷⁶²-Xaa⁷⁶³-Xaa⁷⁶⁴-Xaa⁷⁶⁵-Xaa⁷⁶⁶-Xaa⁷⁶⁷-Xaa⁷⁶⁸,
wherein Xaa⁷⁵⁷ = Ala/Gly, Xaa⁷⁵⁸ = Val/Leu/Ile/Gly/Ala, Xaa⁷⁵⁹ = Tyr/Phe/Trp, Xaa⁷⁶⁰ = Asp/Glu/Ala/Gly, Xaa⁷⁶¹ = Gly/Ala/Lys/Arg/His, Xaa⁷⁶² = Tyr/Phe/Trp/Asp/Glu, Xaa⁷⁶³ = Tyr/Phe/Trp, Xaa⁷⁶⁴ = Pro/Val/Leu/Ile, Xaa⁷⁶⁵ = Phe/Tyr/Trp/Met/Cys, Xaa⁷⁶⁶ = Asp/Glu, Xaa⁷⁶⁷ = Tyr/Phe/Trp, Xaa⁷⁶⁸ = Phe/Tyr/Trp

An example of SEQ ID NO: 307 includes the following SEQ ID NOs: 323, 324 and 364 to 366.

SEQ ID NO: 323: Xaa¹⁹²-Xaa¹⁹³-Xaa¹⁹⁴-Xaa¹⁹⁵-Xaa¹⁹⁶-Xaa¹⁹⁷-Xaa¹⁹⁸-Xaa¹⁹⁹-Xaa²⁰⁰-Xaa²⁰¹-Xaa²⁰²,
wherein Xaa¹⁹² = Ala/Gly, Xaa¹⁹³ = Val/Leu/Ile, Xaa¹⁹⁴ = Tyr/Phe/Trp, Xaa¹⁹⁵ = Asp/Glu, Xaa¹⁹⁶ = Gly/Ala, Xaa¹⁹⁷ = Tyr/Phe/Trp, Xaa¹⁹⁸ = Tyr/Phe/Trp, Xaa¹⁹⁹ = Pro/Val/Leu/Ile, Xaa²⁰⁰ = Phe/Tyr/Trp, Xaa²⁰¹ = Asp/Glu, Xaa²⁰² = Tyr/Phe/Trp

SEQ ID NO: 324: Xaa²⁰³-Xaa²⁰⁴-Xaa²⁰⁵-Xaa²⁰⁶-Xaa²⁰⁷-Xaa²⁰⁸-Xaa²⁰⁹-Xaa²¹⁰-Xaa²¹¹-Xaa²¹²-Xaa²¹³,
wherein Xaa²⁰³ = Ala/Gly, Xaa²⁰⁴ = Gly/Ala, Xaa²⁰⁵ = Tyr/Phe/Trp, Xaa²⁰⁶ = Ala/Gly, Xaa²⁰⁷ = Lys/Arg/His, Xaa²⁰⁸ = Asp/Glu, Xaa²⁰⁹ = Tyr/Phe/Trp, Xaa²¹⁰ = Pro/Val/Leu/Ile, Xaa²¹¹ = Met/Cys, Xaa²¹² = Asp/Glu, Xaa²¹³ = Tyr/Phe/Trp

SEQ ID NO: 364: Xaa⁵³⁵-Xaa⁵³⁶-Xaa⁵³⁷-Xaa⁵³⁸-Xaa⁵³⁹-Xaa⁵⁴⁰-Xaa⁵⁴¹-Xaa⁵⁴²-Xaa⁵⁴³-Xaa⁵⁴⁴-Xaa⁵⁴⁵,
wherein Xaa⁵³⁵ = Gly/Ala, Xaa⁵³⁶ = Lys/Arg/His, Xaa⁵³⁷ = Gln/Asn, Xaa⁵³⁸ = Tyr/Phe/Trp, Xaa⁵³⁹ = Tyr/Phe/Trp, Xaa⁵⁴⁰ = Gly/Ala, Xaa⁵⁴¹ = Val/Leu/Ile, Xaa⁵⁴² = Ser/Thr, Xaa⁵⁴³ = Gly/Ala, Xaa⁵⁴⁴ = Asp/Glu, Xaa⁵⁴⁵ = Tyr/Phe/Trp

SEQ ID NO: 365: Xaa⁵⁴⁶-Xaa⁵⁴⁷-Xaa⁵⁴⁸-Xaa⁵⁴⁹-Xaa⁵⁵⁰-Xaa⁵⁵¹-Xaa⁵⁵²-Xaa⁵⁵³-Xaa⁵⁵⁴-Xaa⁵⁵⁵-Xaa⁵⁵⁶-Xaa⁵⁵⁷-Xaa⁵⁵⁸-Xaa⁵⁵⁹-Xaa⁵⁶⁰-Xaa⁵⁶¹, wherein Xaa⁵⁴⁶ = Gly/Ala, Xaa⁵⁴⁷ = Lys/Arg/His, Xaa⁵⁴⁸ = Lys/Arg/His, Xaa⁵⁴⁹ = Gly/Ala, Xaa⁵⁵⁰ = Val/Leu/Ile, Xaa⁵⁵¹ = Tyr/Phe/Trp, Xaa⁵⁵² = Gln/Asn, Xaa⁵⁵³ = Tyr/Phe/Trp, Xaa⁵⁵⁴ = Asp/Glu, Xaa⁵⁵⁵ = Val/Leu/Ile, Xaa⁵⁵⁶ = Gly/Ala, Xaa⁵⁵⁷ = Ser/Thr, Xaa⁵⁵⁸ = Tyr/Phe/Trp, Xaa⁵⁵⁹ = Tyr/Phe/Trp, Xaa⁵⁶⁰ = Gly/Ala, Xaa⁵⁶¹ = Tyr/Phe/Trp

SEQ ID NO: 366: Xaa⁵⁶²-Xaa⁵⁶³-Xaa⁵⁶⁴-Xaa⁵⁶⁵-Xaa⁵⁶⁶-Xaa⁵⁶⁷-Xaa⁵⁶⁸-Xaa⁵⁶⁹-Xaa⁵⁷⁰-Xaa⁵⁷¹-Xaa⁵⁷²-Xaa⁵⁷³-Xaa⁵⁷⁴-Xaa⁵⁷⁵-Xaa⁵⁷⁶-Xaa⁵⁷⁷-Xaa⁵⁷⁸, wherein Xaa⁵⁶² = Gly/Ala, Xaa⁵⁶³ = Lys/Arg/His, Xaa⁵⁶⁴ = Lys/Arg/His, Xaa⁵⁶⁵ = Gly/Ala, Xaa⁵⁶⁶ = Tyr/Phe/Trp, Xaa⁵⁶⁷ = Lys/Arg/His, Xaa⁵⁶⁸ = Tyr/Phe/Trp, Xaa⁵⁶⁹ = Ser/Thr, Xaa⁵⁷⁰ = Gln/Asn, Xaa⁵⁷¹ = Tyr/Phe/Trp, Xaa⁵⁷² = Gly/Ala, Xaa⁵⁷³ = Cys/Met, Xaa⁵⁷⁴ = Gly/Ala, Xaa⁵⁷⁵ = Tyr/Phe/Trp, Xaa⁵⁷⁶ = Tyr/Phe/Trp, Xaa⁵⁷⁷ = Gly/Ala, Xaa⁵⁷⁸ = Tyr/Phe/Trp

SEQ ID NO: 308: Xaa⁷⁹-Xaa⁸⁰-Xaa⁸¹-Xaa⁸²-Xaa⁸³-Xaa⁸⁴-Xaa⁸⁵-Xaa⁸⁶-Xaa⁸⁷-Xaa⁸⁸-Xaa⁸⁹-Xaa⁹⁰,
wherein Xaa⁷⁹ = Gln/Asn/Ser/Thr/Lys/Arg/His, Xaa⁸⁰ = Ser/Thr/Gly/Ala, Xaa⁸¹ = Leu/Val/Ile, Xaa⁸² = Leu/Val/Ile/Tyr/Phe/Trp/Ser/Thr/deletion, Xaa⁸³ = Asn/Gln/Arg/Lys/His/Ser/Thr/Asp/Glu/deletion, Xaa⁸⁴ = Ser/Thr/Leu/Val/Ile/deletion, Xaa⁸⁵ = Arg/Lys/His/Gly/Ala/Gln/Asn/Asp/Glu/deletion, Xaa⁸⁶ = Thr/Ser/Gly/Ala/deletion, Xaa⁸⁷ = Arg/Lys/His/Gln/Asn/deletion, Xaa⁸⁸ = Lys/Arg/His/Tyr/Phe/Trp/deletion, Xaa⁸⁹ = Asn/Gln/Ser/Thr/deletion, Xaa⁹⁰ = Tyr/Phe/Trp

An example of SEQ ID NO: 308 includes the following SEQ ID NO: 508.

SEQ ID NO: 508: Xaa⁷⁶⁸-Xaa⁷⁶⁹-Xaa⁷⁷⁰-Xaa⁷⁷¹-Xaa⁷⁷²-Xaa⁷⁷³-Xaa⁷⁷⁴-Xaa⁷⁷⁵-Xaa^{77 6}-Xaa⁷⁷⁷ -Xaa⁷⁷⁸-Xaa⁷⁷⁹,
wherein Xaa⁷⁶⁸ = Gln/Asn, Xaa⁷⁶⁹ = Ser/Thr, Xaa⁷⁷⁰ = Leu/Val/Ile, Xaa⁷⁷¹ = Leu/Val/Ile, Xaa⁷⁷² = Asn/Gln, Xaa⁷⁷³ = Ser/Thr, Xaa⁷⁷⁴ = Arg/Lys/His, Xaa⁷⁷⁵ = Thr/Ser, Xaa⁷⁷⁶ = Arg/Lys/His, Xaa⁷⁷⁷ = Lys/Arg/His, Xaa⁷⁷⁸ = Asn/Gln, Xaa⁷⁷⁹ = Tyr/Phe/Trp

An example of SEQ ID NO: 308 includes the following SEQ ID NOs: 367 to 371.

SEQ ID NO: 367: Xaa⁵⁷⁹-Xaa⁵⁸⁰-Xaa⁵⁸¹-Xaa⁵⁸²-Xaa⁵⁸³-Xaa⁵⁸⁴-Xaa⁵⁸⁵-Xaa⁵⁸⁶-Xaa⁵⁸⁷-Xaa⁵⁸⁸-Xaa⁵⁸⁹-Xaa⁵⁹⁰,
wherein Xaa⁵⁷⁹ = Gln/Asn, Xaa⁵⁸⁰ = Ser/Thr, Xaa⁵⁸¹ = Val/Leu/Ile, Xaa⁵⁸² = Val/Leu/Ile, Xaa⁵⁸³ = Gln/Asn, Xaa⁵⁸⁴ = Ser/Thr, Xaa⁵⁸⁵ = Arg/Lys/His, Xaa⁵⁸⁶ = Ser/Thr, Xaa⁵⁸⁷ = Arg/Lys/His, Xaa⁵⁸⁸ = Arg/Lys/His, Xaa⁵⁸⁹ = Gln/Asn, Xaa⁵⁹⁰ = Tyr/Phe/Trp

SEQ ID NO: 368: Xaa⁵⁹¹-Xaa⁵⁹²-Xaa⁵⁹³-Xaa⁵⁹⁴-Xaa⁵⁹⁵-Xaa⁵⁹⁶-Xaa⁵⁹⁷-Xaa⁵⁹⁸-Xaa⁵⁹⁹-Xaa⁶⁰⁰-Xaa⁶⁰¹,
wherein Xaa⁵⁹¹ = Gln/Asn, Xaa⁵⁹² = Gly/Ala, Xaa⁵⁹³ = Val/Leu/Ile, Xaa⁵⁹⁴ = Val/Leu/Ile, Xaa⁵⁹⁵ = Arg/Lys/His, Xaa⁵⁹⁶ = Ser/Thr, Xaa⁵⁹⁷ = Gln/Asn, Xaa⁵⁹⁸ = Gly/Ala, Xaa⁵⁹⁹ = Gln/Asn, Xaa⁶⁰⁰ = Ser/Thr, Xaa⁶⁰¹ = Tyr/Phe/Trp

SEQ ID NO: 369: Xaa⁶⁰²-Xaa⁶⁰³-Xaa⁶⁰⁴-Xaa⁶⁰⁵-Xaa⁶⁰⁶-Xaa⁶⁰⁷-Xaa⁶⁰⁸. Xaa⁶⁰⁹-Xaa⁶¹⁰-Xaa⁶¹¹,
wherein Xaa⁶⁰² = Arg/Lys/His, Xaa⁶⁰³ = Ser/Thr, Xaa⁶⁰⁴ = Val/Leu/Ile, Xaa⁶⁰⁵ = Ser/Thr, Xaa⁶⁰⁶ = Ser/Thr, Xaa⁶⁰⁷ = Ser/Thr, Xaa⁶⁰⁸ = Gly/Ala, Xaa⁶⁰⁹ = Tyr/Phe/Trp, Xaa⁶¹⁰ = Ser/Thr, Xaa⁶¹¹ = Tyr/Phe/Trp

SEQ ID NO: 370: Xaa⁶¹²-Xaa⁶¹³-Xaa⁶¹⁴-Xaa⁶¹⁵-Xaa⁶¹⁶-Xaa⁶¹⁷-Xaa⁶¹⁸. Xaa⁶¹⁹-Xaa⁶²⁰-Xaa⁶²¹-Xaa⁶²²,
wherein Xaa⁶¹² = Asn/Gln, Xaa⁶¹³ = Ser/Thr, Xaa⁶¹⁴ = Val/Leu/Ile, Xaa⁶¹⁵ = Val/Leu/Ile, Xaa⁶¹⁶ = Arg/Lys/His, Xaa⁶¹⁷ = Ser/Thr, Xaa⁶¹⁸ = Asn/Gln, Xaa⁶¹⁹ = Gly/Ala, Xaa⁶²⁰ = Asn/Gln, Xaa⁶²¹ = Ser/Thr, Xaa⁶²² = Tyr/Phe/Trp

SEQ ID NO: 371: Xaa⁶²³-Xaa⁶²⁴-Xaa⁶²⁵-Xaa⁶²⁶-Xaa⁶²⁷-Xaa⁶²⁸-Xaa⁶²⁹-Xaa⁶³⁰-Xaa⁶³¹-Xaa⁶³²-Xaa⁶³³,
wherein Xaa⁶²³ = Asn/Gln, Xaa⁶²⁴ = Gly/Ala, Xaa⁶²⁵ = Val/Leu/Ile, Xaa⁶²⁶ = Val/Leu/Ile, Xaa⁶²⁷ = Arg/Lys/His, Xaa⁶²⁸ = Ser/Thr, Xaa⁶²⁹ = Asn/Gln, Xaa⁶³⁰ = Gly/Ala, Xaa⁶³¹ = Asn/Gln, Xaa⁶³² = Ser/Thr, Xaa⁶³³ = Tyr/Phe/Trp

SEQ ID NO: 309: Xaa⁹¹-Xaa⁹²-Xaa⁹³,
wherein Xaa⁹¹ = Trp/Phe/Tyr/Lys/Arg/His/Gly/Ala/Leu/Val/Ile, Xaa⁹² = Ala/Gly/Leu/Val/Ile/Ser/Thr, Xaa⁹³ = Ser/Thr

An example of SEQ ID NO: 309 includes the following SEQ ID NO: 509.

SEQ ID NO: 509: Xaa⁷⁸⁰-Xaa⁷⁸¹-Xaa⁷⁸²,
wherein Xaa⁷⁸⁰ = Trp/Phe/Tyr, Xaa⁷⁸¹ = Ala/Gly, Xaa⁷⁸² = Ser/Thr

SEQ ID NO: 309 is preferably the following SEQ ID NOs: 372 to 374.

SEQ ID NO: 372: Xaa⁶³⁴-Xaa⁶³⁵-Xaa⁶³⁶,
wherein Xaa⁶³⁴ = Tyr/Phe/Trp, Xaa⁶³⁵ = Ala/Gly, Xaa⁶³⁶ = Ser/Thr

SEQ ID NO: 373: Xaa⁶³⁷-Xaa⁶³⁸-Xaa⁶³⁹,
wherein Xaa⁶³⁷ = Arg/Lys/His, Xaa⁶³⁸ = Val/Leu/Ile, Xaa⁶³⁹ = Ser/Thr

SEQ ID NO: 374: Xaa⁶⁴⁰-Xaa⁶⁴¹-Xaa⁶⁴²,
wherein Xaa⁶⁴⁰ = Leu/Val/Ile, Xaa⁶⁴¹ = Val/Leu/Ile, Xaa⁶⁴² = Ser/Thr

SEQ ID NO: 310: Xaa⁹⁴-Xaa⁹⁵-Xaa⁹⁶-Xaa⁹⁷-Xaa⁹⁸-Xaa⁹⁹-Xaa¹⁰⁰-Xaa¹⁰¹-Xaa¹⁰²,
wherein Xaa⁹⁴ = Gln/Asn/Lys/Arg/His/Tyr/Phe/Trp/Ser/Thr, Xaa⁹⁵ = Gln/Asn/Lys/Arg/His, Xaa⁹⁶ = Ser/Thr/Asp/Glu/Ala/Gly/Tyr/Phe/Trp/Val/Leu/Ile, Xaa⁹⁷ = Tyr/Phe/Trp/Ser/Thr/Ala/Gly/deletion, Xaa⁹⁸ = Asn/Gln/Thr/Ser/Lys/Arg/His/Leu/Val/Ile, Xaa⁹⁹ = Leu/Val/Ile/Tyr/Phe/Trp/Lys/Arg/His/Asp/Glu, Xaa¹⁰⁰ = Pro/Val/Leu/Ile, Xaa¹⁰¹ = Trp/Phe/Tyr/Thr/Ser/deletion, Xaa¹⁰² = Thr/Ser/Lys/Arg/His

An example of SEQ ID NO: 310 includes the following SEQ ID NO: 510.

SEQ ID NO: 510: Xaa⁷⁸³-Xaa⁷⁸⁴-Xaa⁷⁸⁵-Xaa⁷⁸⁶-Xaa⁷⁸⁷-Xaa⁷⁸⁸-Xaa⁷⁸⁹-Xaa⁷⁹⁰-Xaa⁷⁹¹,
wherein Xaa⁷⁸³ = Gln/Asn/Lys/Arg/His, Xaa⁷⁸⁴ = Gln/Asn, Xaa⁷⁸⁵ = Ser/Thr, Xaa⁷⁸⁶ = Tyr/Phe/Trp, Xaa⁷⁸⁷ = Asn/Gln/Thr/Ser, Xaa⁷⁸⁸ = Leu/Val/Ile, Xaa⁷⁸⁹ = Pro/Val/Leu/Ile, Xaa⁷⁹⁰ = Trp/Phe/Tyr, Xaa⁷⁹¹ = Thr/Ser

SEQ ID NO: 310 is preferably the following SEQ ID NOs: 325, 326, 375 and 376.
SEQ ID NO: 325: Xaa²¹⁴-Xaa²¹⁵-Xaa²¹⁶-Xaa²¹⁷-Xaa²¹⁸-Xaa²¹⁹-Xaa²²⁰-Xaa²²¹-Xaa²²²,
wherein Xaa²¹⁴ = Gln/Asn, Xaa²¹⁵ = Gln/Asn, Xaa²¹⁶ = Ser/Thr, Xaa²¹⁷ = Tyr/Phe/Trp, Xaa²¹⁸ = Asn/Gln, Xaa²¹⁹ = Leu/Val/Ile, Xaa²²⁰ = Pro/Val/Leu/Ile, Xaa²²¹ = Trp/Phe/Tyr, Xaa²²² = Thr/Ser

SEQ ID NO: 326: Xaa²²³-Xaa²²⁴-Xaa²²⁵-Xaa²²⁶-Xaa²²⁷-Xaa²²⁸-Xaa²²⁹-Xaa²³⁰-Xaa²³¹,
wherein Xaa²²³ = Lys/Arg/His, Xaa²²⁴ = Gln/Asn, Xaa²²⁵ = Ser/Thr, Xaa²²⁶ = Tyr/Phe/Trp, Xaa²²⁷ = Thr/Ser, Xaa²²⁸ = Leu/Val/Ile, Xaa²²⁹ = Pro/Val/Leu/Ile, Xaa²³⁰ = Trp/Phe/Tyr, Xaa²³¹ = Thr/Ser

SEQ ID NO: 375: Xaa⁶⁴³-Xaa⁶⁴⁴-Xaa⁶⁴⁵-Xaa⁶⁴⁶-Xaa⁶⁴⁷-Xaa⁶⁴⁸-Xaa⁶⁴⁹-Xaa⁶⁵⁰-Xaa⁶⁵¹,
wherein Xaa⁶⁴³ = Ser/Thr/His, Xaa⁶⁴⁴ = Asn/Gln, Xaa⁶⁴⁵ = Ser/Thr, Xaa⁶⁴⁶ = Ser/Thr, Xaa⁶⁴⁷ = Lys/Arg/His, Xaa⁶⁴⁸ = Val/Leu/Ile, Xaa⁶⁴⁹ = Pro, Xaa⁶⁵⁰ = Tyr/Phe/Trp, Xaa⁶⁵¹ = Ser/Thr

SEQ ID NO: 376: Xaa⁶⁵²-Xaa⁶⁵³-Xaa⁶⁵⁴-Xaa⁶⁵⁵-Xaa⁶⁵⁶-Xaa⁶⁵⁷-Xaa⁶⁵⁸-Xaa⁶⁵⁹,
wherein Xaa⁶⁵² = Asn/Gln, Xaa⁶⁵³ = Lys/Arg/His, Xaa⁶⁵⁴ = Val/Leu/Ile, Xaa⁶⁵⁵ = Lys/Arg/His, Xaa⁶⁵⁶ = Asp/Glu, Xaa⁶⁵⁷ = Val/Leu/Ile, Xaa⁶⁵⁸ = Ser/Thr, Xaa⁶⁵⁹ = Lys/Arg/His

### (3) Monoclonal antibody (III)

The monoclonal antibody (III) comprises a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 311; and a light chain variable region comprising a light chain complementarity determining region 1 comprising an amino acid sequence of SEQ ID NO: 312, a light chain complementarity determining region 2 comprising an amino acid sequence of SEQ ID NO: 313, and a light chain complementarity determining region 3 comprising an amino acid sequence of SEQ ID NO: 314; wherein the monoclonal antibody binds to A1 beta-casein and A2 beta-casein.

SEQ ID NO: 311: Xaa¹⁰²-Xaa¹⁰³-Xaa¹⁰⁴-Xaa¹⁰⁵-Xaa¹⁰⁶-Xaa¹⁰⁷-Xaa¹⁰⁸-Xaa¹⁰⁹-Xaa¹¹⁰-Xaa¹¹¹-Xaa¹¹²-Xaa¹¹³-Xaa¹¹⁴,
wherein Xaa¹⁰² = Val/Leu/Ile/Ala/Gly/Ser/Thr, Xaa¹⁰³ = Arg/Lys/His/Ser/Thr, Xaa¹⁰⁴ = His/Lys/Arg/Gly/Ala/Val/Leu/Ile/deletion, Xaa¹⁰⁵ = Gly/Ala/Ser/Thr/deletion, Xaa¹⁰⁶ = Arg/His/Lys/Gly/Ala/deletion, Xaa¹⁰⁷ = Arg/His/Lys/Ser/Thr/Phe/Tyr/Trp/Gly/Ala/deletion, Xaa¹⁰⁸ = Gln/Asn/Pro/deletion, Xaa¹⁰⁹ = Phe/Tyr/Trp/Val/Leu/Ile/deletion, Xaa¹¹⁰ = Asp/Glu/Pro/Ala/Gly/Gln/Asn/Gly/Ala/Phe/Tyr/Trp/deletion, Xaa¹¹¹ = Ser/Thr/deletion, Xaa¹¹² = Phe/Tyr/Trp/Ser/Thr/deletion, Xaa¹¹³ = Arg/His/Lys/Phe/Tyr/Trp/Asp/Glu/deletion, Xaa¹¹⁴ = Val/Leu/Ile/Phe/Tyr/Trp/deletion

An example of SEQ ID NO: 311 includes the following SEQ ID NO: 511.

SEQ ID NO: 511: Xaa⁷⁹²-Xaa⁷⁹³-Xaa⁷⁹⁴-Xaa⁷⁹⁵-Xaa⁷⁹⁶-Xaa⁷⁹⁷-Xaa⁷⁹⁸-Xaa⁷⁹⁹-Xaa⁸⁰⁰-Xaa⁸⁰¹,
wherein Xaa⁷⁹² = Val/Leu/Ile/Ala/Gly, Xaa⁷⁹³ = Arg/Lys/His, Xaa⁷⁹⁴ = His/Lys/Arg, Xaa⁷⁹⁵ = Gly/Ala/deletion, Xaa⁷⁹⁶ = Arg/His/Lys, Xaa⁷⁹⁷ = Arg/His/Lys/Ser/Thr, Xaa⁷⁹⁸ = Gln/Asn/deletion, Xaa⁷⁹⁹ = Phe/Tyr/Trp, Xaa⁸⁰⁰ = Asp/Glu/Pro/Ala/Gly, Xaa⁸⁰¹ = Phe/Tyr/Trp

SEQ ID NO: 311 is preferably the following SEQ ID NOs: 327 to 329 and 377.

SEQ ID NO: 327: Xaa²³²-Xaa²³³-Xaa²³⁴-Xaa²³⁵-Xaa²³⁶-Xaa²³⁷-Xaa²³⁸-Xaa²³⁹-Xaa²⁴⁰,
wherein Xaa²³² = Val/Leu/Ile, Xaa²³³ = Arg/Lys/His, Xaa²³⁴ = His/Lys/Arg, Xaa²³⁵ = Gly/Ala, Xaa²³⁶ = Arg/His/Lys, Xaa²³⁷ = Arg/His/Lys, Xaa²³⁸ = Phe/Tyr/Trp, Xaa²³⁹ = Asp/Glu, Xaa²⁴⁰ = Phe/Tyr/Trp

SEQ ID NO: 328: Xaa²⁴¹-Xaa²⁴²-Xaa²⁴³-Xaa²⁴⁴-Xaa²⁴⁵-Xaa²⁴⁶-Xaa²⁴⁷-Xaa²⁴⁸,
wherein Xaa²⁴¹ = Ala/Gly, Xaa²⁴² = Arg/Lys/His, Xaa²⁴³ = His/Lys/Arg, Xaa²⁴⁴ = Arg/His/Lys, Xaa²⁴⁵ = Ser/Thr, Xaa²⁴⁶ = Phe/Tyr/Trp, Xaa²⁴⁷ = Pro/Ala/Gly, Xaa²⁴⁸ = Phe/Tyr/Trp

SEQ ID NO: 329: Xaa²⁴⁹-Xaa²⁵⁰-Xaa²⁵¹-Xaa²⁵²-Xaa²⁵³-Xaa²⁵⁴-Xaa²⁵⁵-Xaa²⁵⁶-Xaa²⁵⁷-Xaa²⁵⁸,
wherein Xaa²⁴⁹ = Ala/Gly, Xaa²⁵⁰ = Arg/Lys/His, Xaa²⁵¹ = His/Lys/Arg, Xaa²⁵² = Gly/Ala, Xaa²⁵³ = Arg/His/Lys, Xaa²⁵⁴ = Ser/Thr, Xaa²⁵⁵ = Gln/Asn, Xaa²⁵⁶ = Phe/Tyr/Trp, Xaa²⁵⁷ = Ala/Gly, Xaa²⁵⁸ = Phe/Tyr/Trp

SEQ ID NO: 377: Xaa⁶⁶⁰-Xaa⁶⁶¹-Xaa⁶⁶²-Xaa⁶⁶³-Xaa⁶⁶⁴-Xaa⁶⁶⁵-Xaa⁶⁶⁶-Xaa⁶⁶⁷-Xaa⁶⁶⁸-Xaa⁶⁶⁹-Xaa⁶⁷⁰-Xaa⁶⁷¹-Xaa⁶⁷²,
wherein Xaa⁶⁶⁰ = Ser/Thr, Xaa⁶⁶¹ = Arg/Lys/His, Xaa⁶⁶² = Val/Leu/Ile, Xaa⁶⁶³ = Ala/Gly, Xaa⁶⁶⁴ = Ala/Gly, Xaa⁶⁶⁵ = Ala/Gly, Xaa⁶⁶⁶ = Pro, Xaa⁶⁶⁷ = Phe/Tyr/Trp, Xaa⁶⁶⁸ = Phe/Tyr/Trp, Xaa⁶⁶⁹ = Ser/Thr, Xaa⁶⁷⁰ = Phe/Tyr/Trp, Xaa⁶⁷¹ = Glu/Asp, Xaa⁶⁷² = Phe/Tyr/Trp

SEQ ID NO: 312: Xaa¹¹⁵-Xaa¹¹⁶-Xaa¹¹⁷-Xaa¹¹⁸-Xaa¹¹⁹-Xaa¹²⁰-Xaa¹²¹-Xaa¹²²-Xaa¹²³-Xaa¹²⁴-Xaa¹²⁵-Xaa¹²⁶,
wherein Xaa¹¹⁵ = Gln/Asn, Xaa¹¹⁶ = Asn/Gln/Asp/Glu/Ser/Thr/deletion, Xaa¹¹⁷ = Ser/Thr/Ile/Val/Leu/deletion, Xaa¹¹⁸ = Ile/Val/Leu/deletion, Xaa¹¹⁹ = Val/Leu/Ile/Gly/Ala/Tyr/Phe/Trp/Asp/Glu/deletion, Xaa¹²⁰ = His/Lys/Arg/Ser/Thr/Asn/Gln/deletion, Xaa¹²¹ = Ser/Thr/deletion, Xaa¹²² = Asp/Glu/Gly/Ala/deletion, Xaa¹²³ = Gly/Ala/His/Lys/Arg/deletion, Xaa¹²⁴ = Asn/Gln/Asp/Glu/Lys/Arg/His/deletion, Xaa¹²⁵ = Lys/Arg/His/Thr/Ser/deletion, Xaa¹²⁶ = Tyr/Phe/Trp

An example of SEQ ID NO: 312 includes the following SEQ ID NO: 512.

SEQ ID NO: 512: Xaa⁸⁰²-Xaa⁸⁰³-Xaa⁸⁰⁴-Xaa⁸⁰⁵-Xaa⁸⁰⁶-Xaa⁸⁰⁷-Xaa⁸⁰⁸-Xaa⁸⁰⁹-Xaa⁸¹⁰-Xaa⁸¹¹-Xaa⁸¹²-Xaa⁸¹³,
wherein Xaa⁸⁰² = Gln/Asn, Xaa⁸⁰³ = Asn/Gln/deletion, Xaa⁸⁰⁴ = Ser/Thr/deletion, Xaa⁸⁰⁵ = Ile/Val/Leu, Xaa⁸⁰⁶ = Val/Leu/Ile/deletion, Xaa⁸⁰⁷ = His/Lys/Arg/deletion, Xaa⁸⁰⁸ = Ser/Thr/deletion, Xaa⁸⁰⁹ = Asp/Glu/deletion, Xaa⁸¹⁰ = Gly/Ala/deletion, Xaa⁸¹¹ = Asn/Gln, Xaa⁸¹² = Lys/Arg/His/Thr/Ser, Xaa⁸¹³ = Tyr/Phe/Trp

SEQ ID NO: 312 is preferably the following SEQ ID NOs: 330, 331 and 378.

SEQ ID NO: 330: Xaa²⁵⁸-Xaa²⁵⁹-Xaa²⁶⁰-Xaa²⁶¹-Xaa²⁶²-Xaa²⁶³, wherein Xaa²⁵⁸ = Gln/Asn, Xaa²⁵⁹ = Asn/Gln, Xaa²⁶⁰ = Ile/Val/Leu, Xaa²⁶¹ = Asn/Gln, Xaa²⁶² = Lys/Arg/His, Xaa²⁶³ = Tyr/Phe/Trp

SEQ ID NO: 331: Xaa²⁶⁴-Xaa²⁶⁵-Xaa²⁶⁶-Xaa²⁶⁷-Xaa²⁶⁸-Xaa²⁶⁹-Xaa²⁷⁰-Xaa²⁷¹-Xaa²⁷²-Xaa²⁷³-Xaa²⁷⁴ ,
wherein Xaa²⁶⁴ = Gln/Asn, Xaa²⁶⁵ = Ser/Thr, Xaa²⁶⁶ = Ile/Val/Leu, Xaa²⁶⁷ = Val/Leu/Ile, Xaa²⁶⁸ = His/Lys/Arg, Xaa²⁶⁹ = Ser/Thr, Xaa²⁷⁰ = Asp/Glu, Xaa²⁷¹ = Gly/Ala, Xaa²⁷² = Asn/Gln, Xaa²⁷³ = Thr/Ser, Xaa²⁷⁴ = Tyr/Phe/Trp

SEQ ID NO: 378: Xaa⁶⁷³-Xaa⁶⁷⁴-Xaa⁶⁷⁵-Xaa⁶⁷⁶-Xaa⁶⁷⁷-Xaa⁶⁷⁸, wherein Xaa⁶⁷³ = Gln/Asn, Xaa⁶⁷⁴ = Glu/Asp, Xaa⁶⁷⁵ = Val/Leu/Ile, Xaa⁶⁷⁶ = Ala/Gly, Xaa⁶⁷⁷ = Gln/Asn, Xaa⁶⁷⁸ = Phe/Tyr/Trp

SEQ ID NO: 313: Xaa¹²⁷-Xaa¹²⁸-Xaa¹²⁹,
wherein Xaa¹²⁷ = Asn/Gln/Arg/Lys/His/Gly/Ala/Val/Leu/Ile, Xaa¹²⁸ = Thr/Ser/Val/Leu/Ile/His/Lys/Arg/Gly/Ala, Xaa¹²⁹ = Asn/Gln/Ser/Thr

An example of SEQ ID NO: 313 includes the following SEQ ID NO: 513.

SEQ ID NO: 513: Xaa⁸¹⁴-Xaa⁸¹⁵-Xaa⁸¹⁶,
wherein Xaa⁸¹⁴ = Asn/Gln/Arg/Lys/His, Xaa⁸¹⁵ = Thr/Ser/Val/Leu/Ile, Xaa⁸¹⁶ = Asn/Gln/Ser/Thr

SEQ ID NO: 313 is preferably the following SEQ ID NOs: 332, 333 and 379.

SEQ ID NO: 332: Xaa²⁷⁵-Xaa²⁷⁶-Xaa²⁷⁷,
wherein Xaa²⁷⁵ = Asn/Gln, Xaa²⁷⁶ = Thr/Ser, Xaa²⁷⁷ = Asn/Gln

SEQ ID NO: 333: Xaa²⁷⁸-Xaa²⁷⁹-Xaa²⁸⁰,
wherein Xaa²⁷⁸ = Arg/Lys/His, Xaa²⁷⁹ = Val/Leu/Ile, Xaa²⁸⁰ = Ser/Thr

SEQ ID NO: 379: Xaa⁶⁷⁹-Xaa⁶⁸⁰-Xaa⁶⁸¹,
wherein Xaa⁶⁷⁹ = Ala/Gly, Xaa⁶⁸⁰ = Ala/Gly, Xaa⁶⁸¹ = Ser/Thr

SEQ ID NO: 314: Xaa¹³⁰-Xaa¹³¹-Xaa¹³²-Xaa¹³³-Xaa¹³⁴-Xaa¹³⁵-Xaa¹³⁶-Xaa¹³⁷-Xaa¹³⁸,
wherein Xaa¹³⁰ = Cys/Met/Phe/Tyr/Trp/Leu/Val/Ile, Xaa¹³¹ = Gln/Asn, Xaa¹³² = His/Lys/Arg/Ser/Thr/Ala/Gly, Xaa¹³³ = Asn/Gln/Ala/Gly/Asp/Glu/His/Lys/Arg/Ser/Thr, Xaa¹³⁴ = Ser/Thr/His/Lys/Arg/Asn/Gln, Xaa¹³⁵ = Trp/Phe/Tyr/Ala/Gly, Xaa¹³⁶ = Pro/Val/Leu/Ile, Xaa¹³⁷ = Val/Leu/Ile/Pro/Asp/Glu/Trp/Phe/Tyr, Xaa¹³⁸ = Thr/Ser

An example of SEQ ID NO: 314 includes the following SEQ ID NO: 514.

SEQ ID NO: 514: Xaa⁸¹⁷-Xaa⁸¹⁸-Xaa⁸¹⁹-Xaa^{820_}Xaa⁸²¹-Xaa⁸²²-Xaa⁸²³-Xaa⁸²⁴-Xaa⁸²⁵,
wherein Xaa⁸¹⁷ = Cys/Met/Phe/Tyr/Trp/Leu/Val/Ile, Xaa⁸¹⁸ = Gln/Asn, Xaa⁸¹⁹ = His/Lys/Arg/Ser/Thr, Xaa⁸²⁰ = Asn/Gln/Ala/Gly, Xaa⁸²¹ = Ser/Thr/His/Lys/Arg, Xaa⁸²² = Trp/Phe/Tyr, Xaa⁸²³ = Pro/Val/Leu/Ile, Xaa⁸²⁴ = Val/Leu/Ile, Xaa⁸²⁵ = Thr/Ser

SEQ ID NO: 314 is preferably the following SEQ ID NOs: 334, 335 and 380.

SEQ ID NO: 334: Xaa²⁸¹-Xaa²⁸²-Xaa²⁸³-Xaa²⁸⁴-Xaa²⁸⁵-Xaa²⁸⁶-Xaa²⁸⁷-Xaa²⁸⁸-Xaa²⁸⁹,
wherein Xaa²⁸¹ = Cys/Met/Phe/Tyr/Trp, Xaa²⁸² = Gln/Asn, Xaa²⁸³ = His/Lys/Arg, Xaa²⁸⁴ = Asn/Gln, Xaa²⁸⁵ = Ser/Thr, Xaa²⁸⁶ = Trp/Phe/Tyr, Xaa²⁸⁷ = Pro/Val/Leu/Ile, Xaa²⁸⁸ = Val/Leu/Ile, Xaa²⁸⁹ = Thr/Ser

SEQ ID NO: 335: Xaa²⁹⁰-Xaa²⁹¹-Xaa²⁹²-Xaa²⁹³-Xaa²⁹⁴-Xaa²⁹⁵-Xaa²⁹⁶-Xaa²⁹⁷-Xaa²⁹⁸,
wherein Xaa²⁹⁰ = Leu/Val/Ile, Xaa²⁹¹ = Gln/Asn, Xaa²⁹² = Ser/Thr, Xaa²⁹³ = Ala/Gly, Xaa²⁹⁴ = His/Lys/Arg, Xaa²⁹⁵ = Trp/Phe/Tyr, Xaa²⁹⁶ = Pro/Val/Leu/Ile, Xaa²⁹⁷ = Val/Leu/Ile, Xaa²⁹⁸ = Thr/Ser

SEQ ID NO: 380: Xaa⁶⁸²-Xaa⁶⁸³-Xaa⁶⁸⁴-Xaa⁶⁸⁵-Xaa⁶⁸⁶-Xaa⁶⁸⁷-Xaa⁶⁸⁸-Xaa⁶⁸⁹-Xaa⁶⁹⁰,
wherein Xaa⁶⁸² = Val/Leu/Ile, Xaa⁶⁸³ = Gln/Asn, Xaa⁶⁸⁴ = His/Lys/Arg, Xaa⁶⁸⁵ = His/Lys/Arg, Xaa⁶⁸⁶ = Gln/Asn, Xaa⁶⁸⁷ = Trp/Phe/Tyr, Xaa⁶⁸⁸ = Pro, Xaa⁶⁸⁹ = Pro, Xaa⁶⁹⁰ = Thr/Ser

An "antibody" is a glycoprotein produced by a B cell of a lymphocyte and has a function to recognize a molecule such as a specific protein to be bound. An antibody has not only a function to specifically bind to a specific molecule referred to as antigen but also a function to detoxify and remove an antigen-containing factor in cooperation with other biological molecule or cell. An antibody basically has a similar molecular structure. The basic structure of an antibody is a Y-shaped four-chain structure consisting of two light chains and two heavy chains of polypeptide chains. A light chain (L chain) is classified into two types of λ chain and κ chain, and all of antibodies have either of the chains. A heavy chain (H chain) is classified into five types of γ chain, µ chain, α chain, δ chain and ε chain, which have different types of structure, and an antibody is classified into isotypes depending on the kind of a heavy chain. An antibody is a monomer-type immunoglobulin, is composed of two heavy chains (γ chains) and two light chains, and has two antigen-binding sites.

A lower half vertical part in the "Y" shape of an antibody is referred to as an "Fc region", and an upper half "V" shaped part is referred to as a "Fab region". An Fc region has an effector function to initiate a reaction after an antibody binds to an antigen, and a Fab region has a function to bind to an antigen. A Fab region and Fc region of a heavy chain are bound to each other through a hinge part. Papain, which is a proteolytic enzyme and which is contained in papaya, decomposes a hinge part to cut into two Fab regions and one Fc region. The part close to the tip of the "Y" shape in a Fab region is referred to as a "variable region (V region)", since there are various changes in the amino acid sequence in order to bind to various antigens. A variable region of a light chain is referred to as a "VL region", and a variable region of a heavy chain is referred to as a "VH region". An Fc region and the other part in a Fab region other than a V region are referred to as a "constant region (C region)", since there is relatively less change. A constant region of a light chain is referred to as a "CL region", and a constant region of a heavy chain is referred to as a "CH region". A CH region is further classified into three regions of CH1 to CH3. A Fab region of a heavy chain is composed of a VH region and CH1, and an Fc region of a heavy chain is composed of CH2 and CH3. There is a hinge part between CH1 and CH2.

A heavy chain and a light chain respectively contain about 100 or more amino acids mainly involved in binding to an antigen. For example, a heavy chain and a light chain contain variable regions composed of 130 to 140 amino acids. A variable region of a heavy chain is also described as a heavy chain variable region (VH). A variable region of a light chain is also described as a light chain variable region (VL). Each variable region contains complementarity determining regions (CDR) forming an antigen-binding site. VH and VL respectively contain 3 CDR. Specifically, VH contains a CDR 1 (HCDR1), a CDR 2 (HCDR2) and a CDR 3 (HCDR3), and VL contains a CDR 1 (LCDR1), a CDR 2 (LCDR2) and a CDR 3 (LCDR3). The regions other than each of CDR in a heavy chain and a light chain are not restricted and contains an amino acid sequence depending on an animal species and an isotype.

The monoclonal antibody of the present invention can be produced by an ordinary method. For example, first, an animal is immunized with an antigen. When an anti-A1 beta-casein antibody specific to A1 beta-casein is produced, an antigen containing the 67^{th} histidine of A1 beta-casein is used. When an anti-A1/A2 beta-casein antibody specific to both of A1 beta-casein and A2 beta-casein is produced, an antigen which does not contain the 67^{th} amino acid and contains an amino acid sequence common to both of A1 beta-casein and A2 beta-casein is used. The number of amino acid residues contained in an antigen may be adjusted to, for example, 10 or more and 20 or less. An example of an experiment animal includes mouse, rat, guinea pig, rabbit, goat, sheep, donkey and chicken. For example, an injectable preparation containing an antigen may be injected into a tail part or intraperitoneally as an administration embodiment of an antigen.

Then, a hybridoma is prepared by taking out a B cell from a spleen or a lymph node of an immunized animal and fusing the B cell with myeloma. A hybridoma which produces an antibody excellent in a specific binding ability to an antigen is screened among the produced hybridomas by using a plate on which the antigen is immobilized. The hybridoma is grown. The target antibody is purified from a culture fluid of the screened hybridoma.

The binding activity of the monoclonal antibody according to the present invention to A1 beta-casein or both of A1 beta-casein and A2 beta-casein may be separately evaluated by surface plasmon resonance, bio layer interferometry method or the like.

The monoclonal antibody of the present invention can be particularly used for detecting A1 beta-casein, since the monoclonal antibody can specifically bind to A1 beta-casein or both of A1 beta-casein and A2 beta-casein. Specifically, A1 beta-casein is detected by mixing a milk sample and the anti-A1 beta-casein antibody of the present invention to selectively bind the anti-A1 beta-casein antibody to A1 beta-casein.

The term "determining amount" is included in "detection" in this disclosure. For example, an amount of A1 beta-casein in a milk sample can be determined by using the monoclonal antibody of the present invention and milk samples in which an amount or a concentration of A1 beta-casein is known to preliminarily prepare a calibration curve. Hereinafter, a method for detecting A1 beta-casein according to the present invention is described step by step but the present invention is not restricted to the following specific embodiments.

### 1. Step to preliminarily treat sample

A milk sample to be subjected to the detection of A1 beta-casein is preliminarily treated in this step in order to improve the detection sensitivity. This step may be arbitrarily carried out. For example, this step is not needed to be carried out when a milk sample is directly subjected to the measurement. An example of the preliminary treatment includes diluting, defatting and sterilization of a milk sample. The dilution factor may be determined within the range in which the concentration of A1 beta-casein can be determined on the basis of the measured value by ELISA with using a calibration curve. For example, the dilution factor may be adjusted to 1000 times or more and 100000 times or less. PBS can be used for the diluting. A milk sample can be defatted by an ordinary method. The sterilization may be either high temperature sterilization or low temperature sterilization.

### 2. ELISA step

The A1 beta-casein contained in a milk sample is detected by ELISA: Enzyme-Linked Immuno Sorbent Assay in this step. ELISA is mainly classified into direct method, indirect method and sandwich method, and sandwich ELISA method is hereinafter described in terms of high accuracy.

### (1) Step to immobilize capture antibody

A capture antibody is immobilized on a plate or the like in this step. A plate on which an antibody is immobilized is commercially available. Specifically, a solution of a capture antibody is added into each well of a microtiter plate made of polyvinyl chloride or polystyrene, and the plate is incubated and then washed.

The capture antibody may be either of anti-A1 beta-casein antibody or anti-A1/A2 beta-casein antibody. A1 beta-casein can be successfully detected in either case of anti-beta-casein antibody or anti-A1/A2 beta-casein antibody as the capture antibody in accordance with the experimental finding by the inventors of the present invention.

### (2) Step to mix milk sample

Then, the preliminarily treated milk sample is added to the plate on which surface the capture antibody is immobilized. As a result, A1 beta-casein or both of A1 beta-casein and A2 beta-casein contained in the milk sample is specifically bound to the present invention antibody. Next, the plate may be washed.

### (3) Detection step

Then, the anti-A1 beta-casein antibody as a detector antibody is added to the plate after the milk sample is added to the plate in order to determine an amount of the detector antibody by an enzyme reaction. The epitope of the detector antibody is different from the epitope of the capture antibody. A capture antibody labeled by an enzyme may be used, or a secondary antibody which specifically binds to a detector antibody and is labeled by an enzyme may be used in addition to a detector antibody.

An amount of the detector antibody may be determined by the method depending on the detector antibody or the labeling enzyme bound to the secondary antibody. For example, when the labeling enzyme is horseradish peroxidase (HRP), hydrogen peroxide and a coloring substance are added. An example of the coloring substance used with HRP includes 3,3',5,5'-tetramethylbenzidine (TMB), o-phenylenediamine dihydrochloride (OPD) and 2,2'-azino-bis[3-ethyl-benzothiazoline-6-sulfonic acid] diammonium salt (ABTS). The absorbance of the wavelength depending on the coloring substance may be measured. For example, when the labeling enzyme is alkaline phosphatase (ALP), nitrophenylphosphate (pNPP) is used as the coloring substance and the absorbance of 405 nm is measured.

An amount of A1 beta-casein in a milk sample can be determined by using milk samples in which the amounts or the ratios of A1 beta-casein are known to prepare a calibration curve as described above.

It is taken for granted that a ratio of A1 beta-casein in beta-casein is 30 mass% or less in A2 milk in which a ratio of A1 beta-casein is low and which is rarely harmful due to less A1 beta-casein. The method for detecting A1 beta-casein according to the present invention is very excellent as an evaluation method for milk, since 2 mass% or more of A1 beta-casein in all beta-casein may be detected by using the monoclonal antibody of the present invention in accordance with the experimental finding by the inventors of the present invention. The ratio is preferably 5 mass% or more, more preferably 10 mass% or more, and even more preferably 20 mass% or more or 30 mass% or more, since A1 beta-casein can be detected more easily in the case where the ratio of A1 beta-casein in all beta-casein is larger.

The present application claims the benefit of the priority date of Japanese patent application No. 2022-158177 filed on September 30, 2022. All of the contents of the Japanese patent application No. 2022-158177 filed on September 30, 2022, are incorporated by reference herein.

### EXAMPLES

Hereinafter, the examples are described to demonstrate the present invention more specifically, but the present invention is in no way restricted by the examples, and the examples can be appropriately modified to be carried out within a range that adapts to the contents of this specification. Such a modified example is also included in the technical range of the present invention.

### Example 1: Preparation of monoclonal antibody by lymph node method

### (1) Preparation of antigen/adjuvant emulsion

Two 1 mL luer lock glass syringes were connected at both ends of a locking needle connector. The plunger was removed from the upper syringe before the syringes were connected. Complete Freund's adjuvant (750 µL) was mixed well and then added into the upper syringe without the plunger using a pipette for non-aqueous liquid. The plunger of the lower syringe was drawn for filling the inside with the adjuvant, and then the plunger was placed back to return the adjuvant into the upper syringe.

The antigen protein shown in Table 1 was dissolved in PBS to prepare 0.5 mg/mL solutions for a mouse and 1 mg/mL solutions for a rat. Each solution (350 µL) was added into the upper syringe, and the plunger of the lower syringe was drawn in order to suck the antigen and the adjuvant into the lower syringe. The upper syringe was removed, and the plunger was placed back. The plunger of the lower syringe was slowly pushed up to remove bubbles.

**Table 1**

| | |
|---|---|
| **A1 beta-casein peptide** | **F⁶²PGPIH⁶⁷NSLP⁷¹** |
| **A2 beta-casein peptide** | **F⁶²PGPIP⁶⁷NSLP⁷¹** |
| **A1/A2 beta-casein common peptide** | **E¹⁴SLSSSEESITRINKKIEK³²** |

The upper syringe was connected, and the syringes were maintained in a horizontal position. The plungers were promptly moved back and forth about 10 times in order to emulsify the mixture. The thus obtained emulsion was drawn into the one syringe. The other syringe and the needle connector were removed, and a disposable needle was attached.

### (2) Injection into muscle of tail root part

A mouse or a rat was anesthetized with sevoflurane, and it was confirmed by the lack of response to stimulation that the mouse and rat were anesthetized. The antigen-adjuvant emulsion was intramuscularly injected at the right side of the tail root part in the volume of 50 µL in the case of a mouse or 100 µL in the case of a rat. The emulsion was similarly injected at the left side 5 seconds after the needle was withdrawn. The injected mouse or rat was encased in a cage at 37°C until the mouse and rat were awakened, and then returned to a normal breeding environment.

### (3) Collection of iliac lymph node

When the injection was successful, the hypertrophied iliac lymph node was collected about 14 to 21 days after the injection. It is expected that 0.5 to 1.5 × 10⁸ of B cells are collected from 6 mice and 0.5 to 2.0 × 10⁸ of B cells are collected from 3 rats. More than 0.5 × 10⁸ of cells are required for electrical cell fusion.

Specifically, the immunized mouse or rat was humanely killed, and 70% ethanol was sprayed on the ventral skin. The ventral skin and muscle were cut open, and the hypertrophied iliac lymph node was found to be taken out with avoiding the intestine. The iliac lymph node was added into Dulbecco's Modified Eagle Medium (DMEM) and transferred under a tissue culture hood.

### (4) Isolation of B cell

The collected lymph node was placed on a steel wire mesh in a 60 mm culture dish, and DMEM (10 mL) was added thereto. The lymph node was broken by pushing the lymph node through the wire mesh with using a spatula. The cell, mainly B cell, was collected in a 50 mL tube with using a pipette. The cell was centrifuged at 1500 rpm and 500 G for 5 minutes, and the supernatant was removed from the cell pellet. The cell was re-suspended in DMEM (10 mL). The centrifugation and the re-suspension were repeated.

### (5) Cell fusion

SP2/0-Ag14 myeloma cell was divided using fresh culture medium 2 days before the fusion in order to lead the cell into logarithmic growth phase, and added into DMEM. The mixture was centrifuged at 1500 rpm for 5 minutes to remove the supernatant from the cell pellet. The cell was re-suspended in DMEM (10 mL). The centrifugation and the re-suspension were repeated. The number of the myeloma cell was counted using a cell counting chamber. A suspension of 2.0 × 10⁷ cells was prepared in a new 50 mL tube.

In addition, the number of the B cell was counted using a cell counting chamber. A suspension of 5.0 × 10⁷ cells was prepared in the other 50 mL tube and incubated at room temperature for 5 minutes.

The B cell suspension and the myeloma cell suspension were mixed, and the mixture was centrifuged at 1500 rpm for 5 minutes. The supernatant was removed from the cell pellet, and the cell was re-suspended in ECF buffer (10 mL). The centrifugation and the re-suspension were repeated. The suspension of both of the cells was centrifuged at 1500 rpm for 5 minutes. The supernatant was removed from the cell pellet, and the cell was re-suspended in ECF buffer (1 mL).

The suspension (500 µL) of both of the cells was added into an electrode chamber, and the chamber was installed at a cell fusion generator ("Super Electro Cell Fusion Generator ECFG21" manufactured by Nepa Gene). The impedance was measured using the Electro Cell Fusion Generator, DC pulse voltage was set, and rectangular pulse was applied. The other cell suspension was repeatedly subjected to the fusion procedure. The fusion cell suspension was collected in a 50 mL tube containing HAT medium (40 mL).

The fusion cell suspension (100 µL) was seeded on a 96-well plate using a multichannel pipette and incubated at 37°C in a CO₂ incubator.

### (6) Cultivation and cryopreservation of hybridoma

The medium was carefully removed using an 8-chanel aspirator 5 days after the cell fusion, and HAT medium (200 µL) was added to each well of the plate.

The well which had a positive response to A1 beta-casein and a negative response to A2 beta-casein or the well which had a positive response to A1 beta-casein and a positive response to A2 beta-casein was identified using the supernatant (100 µL) by ELISA 8 days after the cell fusion.

The cell in the above-described well was transferred to a 24-well plate containing HT medium (1 mL) 1 day after the identification by ELISA. The culture supernatant was collected from each well to a new 1.5 mL tube 2 to 3 days later and preserved at 4°C or -30°C.

New medium (250 µL) was added, and the cell was suspended using a pipette. A preservation medium (250 µL) was added and mixed well. The mixture was transferred into a tube for cryopreservation and preserved at -80°C.

### (7) Cloning of hybridoma

The frozen hybridoma stock was taken out from the freezer of -80°C and rapidly thawed at 37°C. After the cell was completely thawed, a vial in which there was the cell was placed in a tissue culture hood. The culture medium in the vial was exchanged for HT medium, and then the number of the alive hybridoma was counted using a cell counter. A suspension in which 2.0 × 10² cells were dispersed in HT medium (20 mL) was prepared in a 50 mL tube. The thus obtained hybridoma suspension (100 µL) was seeded on two 96-well plates and incubated at 37°C under CO₂. HT medium (100 µL) was added 5 days after the seeding, and the positive well was identified by ELISA 8 days after the seeding.

The positive hybridoma was transferred to a 24-well plate 1 day after the ELISA and continuously cultivated for 2 to 3 days. A preservation medium (250 µL) was added and mixed well, and the mixture was transferred into a cryotube to be stored at -80°C. The positive clone hybridoma was chewed and cultivated until the amount of the hybridoma becomes 3 to 4 plates of 75 cm² in order to produce a large amount of the monoclonal antibody. The culture supernatant containing the monoclonal antibody was collected at logarithmic growth phase.

### (8) Purification of monoclonal antibody

PBS (500 µL) was added into an empty column and discharged therefrom. Then, approximately equivalent amount of an affinity carrier for antibody purification ("Protein G Sepharose 4 Fast Flow" manufactured by GE Healthcare) was mixed well and added thereto. Next, the column was washed once with 0.1 M glycine-HCl (pH 2.0, 2.5 mL) and 2 times with PBS (5 mL).

One twentieth amount of 1 M Tris (pH 8.0) was added to the culture supernatant of the positive clone. A 15 mL tube was placed under the column, and approximately half quantity of the mixture was added into the column and the remaining half quantity was added after the mixture was discharged in order to bind the antibody to Protein G more surely. Then, the column was washed three times with PBS (5 mL). A 5 mL tube to which 1 M Tris (pH 8.0, 500 µL) was added was placed under the column. The antibody was eluted by adding 0.1 M glycine-HCl (pH 2.0, 2.5 mL), and then the eluate was immediately mixed well and transferred to a concentration tube. The eluate was centrifuged while watching the situation until the eluate was concentrated to about 500 µL. The lower phase was abandoned, and PBS (about 5 mL) was added to the upper phase. The mixture was mixed well with turnover and further centrifuged until the amount became about 300 µL. The concentrate was transferred into a 1.5 mL tube, and PBS was added thereto so that the total amount became about 500 µL. The antibody concentration was determined by electrophoresis.

### (9) Amino acid sequence of monoclonal antibody

The total RNA was extracted from the positive clone, and cDNA was produced from the total RNA using a reagent composition for producing cDNA ("SuperScript III First-Strand Synthesis SuperMix" manufactured by Thermo Fisher SCIENTIFIC). The CDR gene was amplified from the cDNA by reverse transcription-PCR using high speed enzyme ("KOD-Plus-Neo" manufactured by TOYOBO) and the primers for the antigen complementarity determining region (CDR) of the antibody, and the nucleotide sequence and the amino acid sequence of the CDR were determined by outsourcing.

The amino acid sequence of the CDR of the anti-A1 beta-casein antibody derived from a rat is shown in Table 2, the amino acid sequence of the CDR of the anti-A1/A2 beta-casein antibody derived from a rat is shown in Table 3, and the amino acid sequence of the CDR of the anti-A1 beta-casein antibody derived from a mouse is shown in Table 4.

**Table 2**

| Anti-A1 beta-casein antibody (I) derived from rat | | | | | | | |
|---|---|---|---|---|---|---|---|
| No. | Subclass | VH | | | VL | | |
| | | CDR1 | CDR2 | CDR3 | CDR1 | CDR2 | CDR3 |
| #5-7 | IgG2c | GFTFSNYG | ISYDGSST | ARPYNNYGYWYFDF | QNIYKN | NAN | QQYYSGLT |
| #13-1 | IgG2c | GFTFSNYG | ISYDGSST | ARPYNNYGYWYFDF | QNIYKN | NAN | QQYYSGLT |
| #14-6 | IgG2c | GFTFSNYG | ISYDGSST | ARPYNNYGYWYFDF | QNIYKN | NAN | QQYYSGLT |
| #4-2 | IgG2b | GFTFSSYG | INYDGSST | ARHGYYDGSYYYGGDFDY | SSVSY | DTS | QQWSNTPYT |
| #17-2 | IgG2a | GFTFSNYG | INYDGSST | ARHSYYDGNYYYGGSFDY | SRVSY | DTS | QQWSSSPYT |

**Table 3**

| Anti-A1/A2 beta-casein antibody (III) derived from rat | | | | | | | |
|---|---|---|---|---|---|---|---|
| No. | Subclass | VH | | | VL | | |
| | | CDR1 | CDR2 | CDR3 | CDR1 | CDR2 | CDR3 |
| #27-1 | IgG2b | GFTFSNYD | ITTSGGST | VRHGRRFDY | QNINKY | NTN | CQHNSWPLT |
| #30-5 | IgG2b | GFTFSNYD | ITTSGGST | ARHRSFPY | QNINKY | NTN | FQHNSWPLT |
| #9-1 | IgG1 | GFTFSNYD | ISPSGGSI | ARHGHTQFAY | QSLVHSDGNTY | RVS | LQSAHFPLT |
| #18-3 | IgG1 | GFTFSNYD | ISPSGGSI | ARHGHTQFAY | QSLVHSDGNTY | RVS | LQSAHFPLT |
| #37-2 | IgG1 | GFTFSNYD | ISPSGGSI | ARHGHTQFAY | QSLVHSDGNTY | RVS | LQSAHFPLT |
| #35-5 | IgG1 | GFTFRNYD | ISPSGGSI | ARHGHTQFAY | QSLVHSDGNTY | RVS | LQSAHFPLT |

**Table 4**

| Anti-A1 beta-casein antibody (II) derived from mouse | | | | | | | |
|---|---|---|---|---|---|---|---|
| No. | Subclass | VH | | | VL | | |
| | | CDR1 | CDR2 | CDR3 | CDR1 | CDR2 | CDR3 |
| m6-3 | IgG2a | GYTFTTYT | INPSSGYI | AVYDGYFPFDY | QSLLNSRTRKNY | WAS | QQSYNLPWT |
| m1-1 | IgG1 | GYTFTSYT | INPSSGYT | AGFAKDYPMDY | QSLLNSRTRKNY | WAS | KQSYTLPWT |
| m21-8 | IgG1 | GYTFTSYT | FNPSSGYT | AVYDGYFPFDY | QSLLNSRTRKNY | WAS | QQSYNLPWT |
| m108-1-1 | IgG2b | GYIFRNYG | INTYTGDP | ARNYYGISGDY | QGLVHSNGNTY | KVS | SQSTHIPYT |

### Example 2: Determination of amount of A1 beta-casein by sandwich ELISA

A1 milk containing A1 beta-casein only and A2 milk containing A2 beta-casein only were respectively diluted 100 times, and the diluted milks were mixed at the composition shown in Table 5 to prepare A1/A2 beta-casein defatted milk.

**Table 5**

| A1 concentration | A2 concentration | A1: A2 |
|---|---|---|
| 100 ng/µL | 0 ng/µL | 1 : 0 |
| 50 ng/µL | 50 ng/µL | 1 : 1 |
| 25 ng/µL | 75 ng/µL | 1 : 3 |
| 10 ng/µL | 100 ng/µL | 1 : 10 |
| 3.3 ng/µL | 100 ng/µL | 1 : 30 |
| 2 ng/µL | 100 ng/µL | 1 : 50 |
| 1 ng/µL | 100 ng/µL | 1 : 100 |
| 0 ng/µL | 100 ng/µL | 0 : 1 |

The anti-A1/A2 beta-casein antibody #27-1 as a capture antibody was diluted with PBS to prepare a 1.5 µg/100 µL solution, and the solution was added to a 96-well plate in the amount of 100 µL/well. The plate was covered with a lid and incubated at 37°C overnight. Then, the liquid part was abandoned, the plate was washed with PBS (200 µL) 2 times, and 1% BSA/PBS (200 µL) was added thereto. The plate was covered with a lid and incubated at 37°C for 1 hour or at 4°C overnight. Then, the liquid part was abandoned, the plate was washed with PBS (200 µL) 2 times, and then the milk sample diluted with PBS 100 times (100 µL) was added thereto. The plate was covered with a lid and incubated at 37°C for 1.5 to 2 hours. Next, the liquid part was abandoned, and the plate was washed with PBS (200 µL) three times.

Then, the anti-A1 beta-casein antibody #6-3 as a detector antibody was diluted with 1% BSA/PBS to prepare a 1.5 µg/100 µL solution. The thus obtained solution (100 µL) was added to the plate. The plate was covered with a lid and incubated at 37°C for 1.5 to 2 hours. The liquid part was abandoned, and the plate was washed with PBS (200 µL) three times.

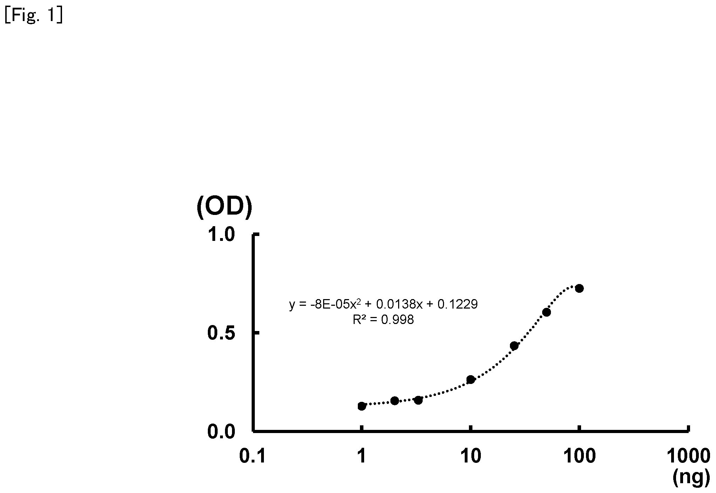

HRP-labeled secondary antibody was diluted 20,000 times with 1% BSA/PBS, and the thus obtained diluted solution (100 µL) was added. The plate was covered with a lid and incubated at room temperature for 1 hour. The liquid part was abandoned, and the plate was washed with PBS (200 µL) three times. A PBS solution of 0.4 mg/mL o-phenylenediamine dihydrochloride + 0.012% hydrogen peroxide was separately prepared as a coloring reagent, and 150 µL of the solution was added to the plate. The plate was stood still at room temperature for 30 to 60 minutes. Then, the reaction was stopped by adding 3 M sulfuric acid (50 µL), and the adsorbent at 492 nm was measured. The adsorbent was measured three times in the same day, and the average value of the measured optical density (OD) value was calculated. The result is shown in Figure 1. The abscissa axis of Figures 1 to 3 is the A1 beta-casein amount per 1 µL of the milk sample before the dilution.

An approximate expression of y = -8×10⁻⁵x² + 0.0138x + 0.1229 was obtained on the basis of the correlation between a content amount of A1 beta-casein and a measured OD value, and the determination coefficient R² thereof was very high as 0.998, as the result shown in Figure 1. In addition, though the existence of A2 beta-casein interferes with the measurement of A1 beta-casein, the OD value could be measured with a sufficient significant difference in comparison with the measured OD value of the sample which did not contain A1 beta-casein even when the sample contained relatively large amount of A2 beta-casein, specifically, when A1 : A2 was 1 : 30 to 50. Since A2 milk of which influence by A1 beta-casein is reduced generally means a milk in which a ratio of A1 beta-casein among beta-casein is reduced to 30% or less, it was demonstrated that the beta-casein contained in milk can be sufficiently evaluated by the present invention. The milk to be actually measured should be defatted and then diluted 10000 times in order to be measured by ELISA in the above-described condition on the basis of the beta-casein concentrations in the above measured samples.

### Example 3: Determination of amount of A1 beta-casein by sandwich ELISA

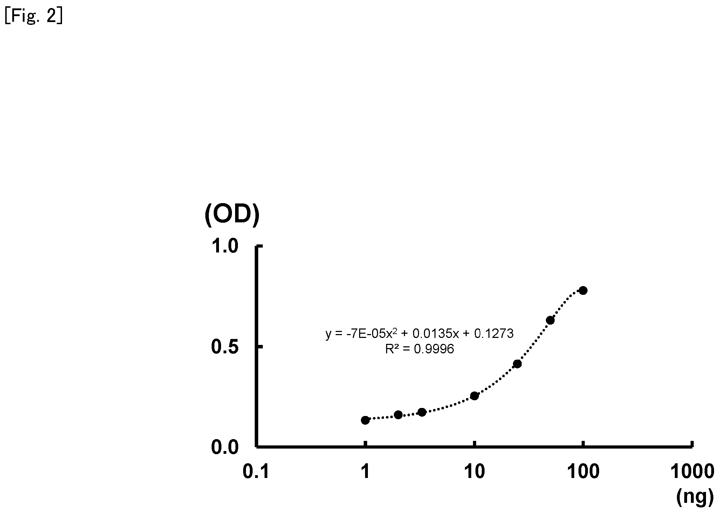

ELISA measurement was carried out similarly to Example 2 except that anti-A1 beta-casein antibody #17-2 was used as a capture antibody in place of anti-A1/A2 beta-casein antibody and anti-A1 beta-casein antibody #6-3 was used as a detector antibody. The result is shown in Figure 2.

Even when the anti-A1 beta-casein antibody was used as a capture antibody, a similar result to the case of the anti-A1/A2 beta-casein antibody was used was obtained as the result shown by Figure 2. Specifically, an approximate expression of y = -7×10⁻⁵x² + 0.0135x + 0.1273 was obtained on the basis of the correlation between a content amount of A1 beta-casein and a measured OD value, and the determination coefficient R² thereof was very high as 0.9996. In addition, it was demonstrated that A1 beta-casein can be detected in a milk sample containing A2 beta-casein even by the combination anti-A1 beta-casein antibody #17-2 and anti-A1 beta-casein antibody #6-3 as a curve very similar to that in Example 2 was prepared.

### Example 4: Determination of amount of A1 beta-casein by sandwich ELISA

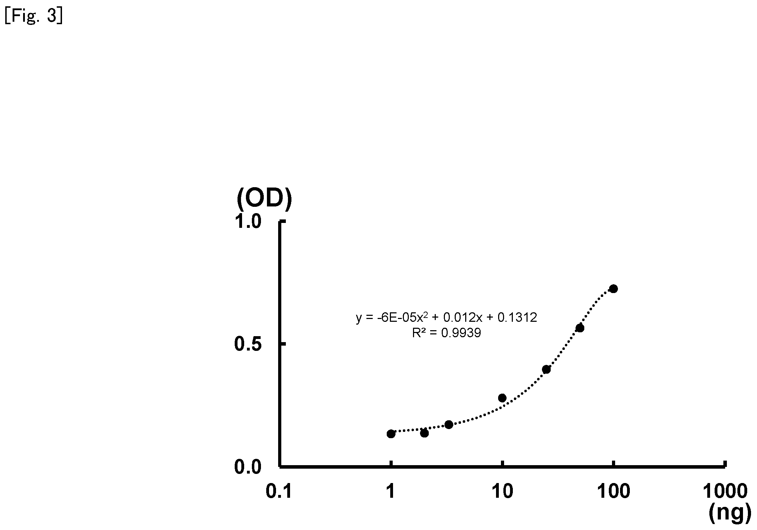

ELISA measurement was carried out similarly to Example 2 except that anti-A1 beta-casein antibody rHis#4-2 was used as a capture antibody and anti-A1 beta-casein antibody mHis#21-8 was used as a detector antibody. The result is shown in Figure 3.

A similar result to the cases of Examples 2 and 3 was obtained as the result shown by Figure 3. Specifically, an approximate expression of y = -6×10⁻⁵x² + 0.012x + 0.1312 was obtained on the basis of the correlation between a content amount of A1 beta-casein and a measured OD value, and the determination coefficient R² thereof was very high as 0.9939. In addition, a curve very similar to that in Examples 2 and 3 was prepared. A1 beta-casein could be detected in a milk sample which contained 200 ng/uL or more A1 beta-casein before the dilution and in a milk sample which contained 2 × 10⁻² ng/µL or more A1 beta-casein after 10000 times dilution in Examples 2 and 3; on the one hand, A1 beta-casein could be detected from the measured OD value in a milk sample which contained 1000 ng/µL or more A1 beta-casein before the dilution and in a milk sample which contained 1 × 10⁻¹ ng/µL or more A1 beta-casein after 10000 times dilution in this Example.

### Example 5: Determination of amount of A1 beta-casein by sandwich ELISA

A1 milk containing A1 beta-casein only and A2 milk containing A2 beta-casein only were respectively diluted 100 times and mixed at the composition shown in Table 6 in order to prepare Al/A2 beta-casein milk. The milk samples were directly used without defatting.

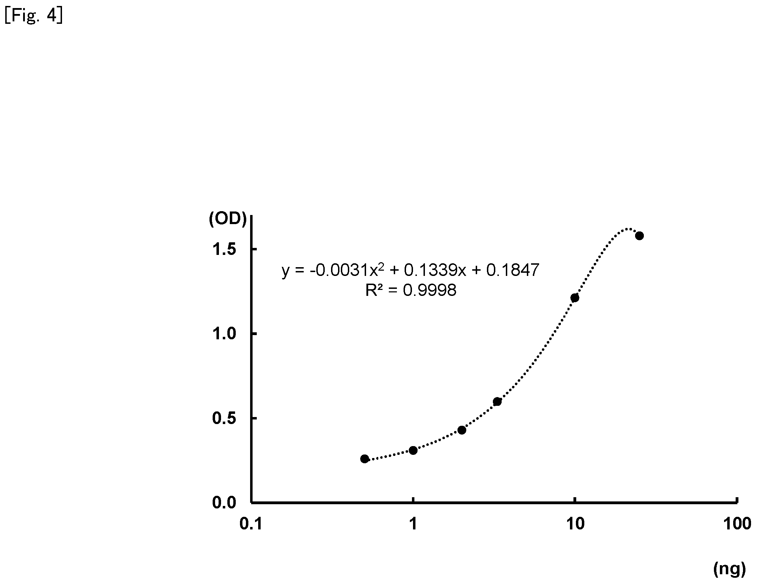

ELISA measurement was carried out similarly to Example 2 except that anti-A1 beta-casein antibody mHis#6-3 was used as a capture antibody and the milk samples without defatting were used. The result is shown in Table 6 and Figure 4.

**Table 6**

| A1 concentration | A1: A2 | OD |
|---|---|---|
| 25 ng/µL | 1 : 3 | 1.579 |
| 10 ng/µL | 1 : 10 | 1.212 |
| 3.3 ng/µL | 1 : 30 | 0.597 |
| 2 ng/µL | 1 : 50 | 0.430 |
| 1 ng/µL | 1 : 100 | 0.311 |
| 0.5 ng/µL | 1 : 200 | 0.260 |
| 0 ng/µL | 0 : 1 | 0.215 |

An approximate expression of y = -3.1×10⁻³x² + 0.1339x + 0.1847 was obtained on the basis of the correlation between a content amount of A1 beta-casein and a measured OD value, and the determination coefficient R² thereof was very high as 0.9998, as the result shown in Table 6 and Figure 4. In addition, it was demonstrated that not only defatted milk but also raw milk can be subjected to the measurement. Furthermore, it was demonstrated that 1/200 times or more by mole of A1 beta-casein to A2 beta-casein in raw milk can be detected by the present invention.

### Example 6: Determination of amount of A1 beta-casein by sandwich ELISA

A1 milk containing A1 beta-casein only and A2 milk containing A2 beta-casein only were respectively diluted 100 times and mixed at the composition shown in Table 6 in order to prepare Al/A2 beta-casein milk. The milk samples were directly used without defatting.

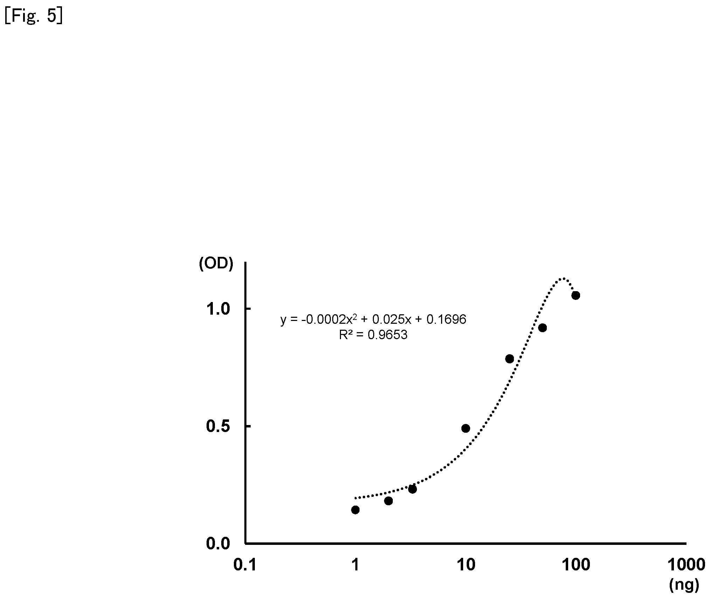

ELISA measurement was carried out similarly to Example 2 except that anti-A1 beta-casein antibody mHis#6-3 was used as a detector antibody and the milk samples without defatting were used. The result is shown in Table 7 and Figure 5.

**Table 7**

| A1 concentration | A1 : A2 | OD |
|---|---|---|
| 100 ng/µL | 1 : 0 | 1.057 |
| 50 ng/µL | 1 : 1 | 0.919 |
| 25 ng/µL | 1 : 3 | 0.787 |
| 10 ng/µL | 1 : 10 | 0.491 |
| 3.3 ng/µL | 1 : 30 | 0.233 |
| 2 ng/µL | 1 : 50 | 0.183 |
| 1 ng/µL | 1 : 100 | 0.145 |
| 0 ng/µL | 0 : 1 | 0.118 |

An approximate expression of y = -2.0×10⁻⁴x² + 0.025x + 0.1696 was obtained on the basis of the correlation between a content amount of A1 beta-casein and a measured OD value, and the determination coefficient R² thereof was very high as 0.9653, as the result shown in Table 7 and Figure 5. In addition, it was demonstrated that not only defatted milk but also raw milk can be subjected to the measurement. Furthermore, it was demonstrated that 1/100 times or more by mole of A1 beta-casein to A2 beta-casein in raw milk can be detected by the present invention.

### Example 7

Anti-A1 beta-casein antibody (I) derived from a rat, anti-Al/A2 beta-casein antibody (III) derived from a rat and anti-A1 beta-casein antibody (II) derived from a mouse which had the following CDR amino acid sequences were prepared. The following each antibodies have an excellent selective binding ability to A1 beta-casein or both of A1 beta-casein and A2 beta-casein.

**Table 8**

| Anti-A1 beta-casein antibody (I) derived from rat | | | | | | | |
|---|---|---|---|---|---|---|---|
| No. | Subclass | VH | | | VL | | |
| | | CDR1 | CDR2 | CDR3 | CDR1 | CDR2 | CDR3 |
| #22-1-12 | IgG2c | GFTFSNYG | ISYDGSST | ARPYNNYGYWYFDF | QNIYKN | NAN | QQYYSGLT |
| #66-1 | IgG2c | GFTFSNYD | ISARSGST | ARGAFFDY | QSLLHSSGNTY | LVS | VQSTHAPWT |
| #68-1 | IgG2c | GFTFSNYD | ISARSGST | ARGAFFDY | QSLLHSSGNTY | LVS | VQSTHAPWT |
| #55-1 | IgG2a | GFTFSNYD | IRYDGGST | TTGYGSTGY | QSLLHSNGNTY | LVS | VQGTHAPPWT |
| #20-2 | IgG2a | GFTFSNYG | ISYDGSST | ARPYYYDGSYYRYYWFFDF | KSISKY | SGS | QQHNEYPPT |
| #113-1 | IgG2a | GYTFTDHA | INTYTGKP | ARGGIIRGTLFDY | QNIYKN | NAN | HHHYSGYT |
| #38-31 | IgG2a | GFTFKNYG | ISYDGSRT | ARPFYNNYYWYDF | QNINKY | NPN | FQHNSGNT |
| #2-23 | IgG2a | GLTFRNYG | ISYDGSGT | ARHGHYDGNYNYGGHFDY | SSVSY | DTS | QQWSSSPYT |
| #3-6 | IgG2b | GFSFSNYG | ISYDGSST | ARHGYYDGSYYYGGDFDY | SSVSY | DTS | QQWSNSPYT |
| #9-4 | IgG2b | GFTFSNYG | ISYDGSST | ARHGYYDGSYYYGGDFDY | SSVSY | DTS | QQWSNTPYT |
| #39-19 | IgG2b | GFTFSNYG | ISYDGSRT | ARHGYYDGSYYYGGDFDY | SSVSY | EIS | QQWSNTPYT |
| #16-2 | IgG2b | GFTFSNYG | LSYDGSGT | ARHGFYDGNYYYGGSFDY | SSVRY | DTS | QQWSSAPYT |
| #34-2 | IgG2b | GFTFSNYG | ISYDGIRT | ARPYSNRYWYFDF | KSISKY | SGS | QQHNEYPPT |
| #10-1 | IgG2b | GFTFSNYG | ISYDGSNT | ARPYSNRYWYFDF | KSISKY | SGS | QQHNEYPPT |
| #108-1 | IgG2b | GFTFSDYW | IKYDGSYT | TRGNSAGDY | KSLLYSNGKTY | WMS | QQFLEYPLT |
| #101-1 | IgG2b | GFTFNNYW | ITNGGGST | TRDRGHWVFDY | QNINNY | NAN | QQYNSWIT |
| #79-1 | IgG2b | GFTFNNYW | ITNGGGST | TRDRGHWVFDY | QNINNY | NAN | QQYNSWIT |
| #83-1 | IgG2b | GFTFNNYW | ITNGGGST | TRDRGHWVFDY | QNINNY | YAN | QQYNSWIT |
| #75-1 | IgG2b | GFTFSNYW | LTNGGGST | SRDRGHWVFDY | QNINNY | YAS | QQYNSWIT |
| #84-1 | IgG2b | GFTFNNYW | LTDGGGST | SRDRGHWVFDY | QNINNY | YAS | QQYNTWIT |
| #73-1 | IgG2b | GYTFTNHY | IGPTNGVT | AREGDYGGYSEGPFDY | QNVDYYGNSY | LAS | QQGRRLPYT |
| #74-1 | IgG2b | GYTFTNHY | IGPTKGVT | AREGDYGGYSEGPFDY | QNVDYYGNSY | LAS | QQGRRLPYT |
| #97-1 | IgG2b | GYTFTNHY | IGPTKGVT | AREGDYGGYSEGPFDY | QNVDYYGNSY | LAS | QQGRRLPYT |
| #56-1 | IgG2b | GLTFSNYG | ISIRGGST | TTGFAY | QSLVYSDGKTY | QVS | VQTTHFPLT |
| #96-1 | IgG2b | GLTFSNYG | ISTRGGST | TTGFAY | QSLVYSDGKTY | QVS | VQTTHFPLT |
| #103-1 | IgG2b | GLIFRNYG | ISTSGGST | TTGFAY | QSLVYSDGKTY | QVS | VQTTHFPLT |
| #109-1 | IgG2b | GYTFTSNF | IYPGDGDT | ASRSGHNNYEGWFAY | QSLVYSDGKTY | QVS | VQTTHFPLT |

**Table 9**

| Anti-A1 /A2 beta-casein antibody (III) derived from rat | | | | | | | |
|---|---|---|---|---|---|---|---|
| No. | Subclass | VH | | | VL | | |
| | | CDR1 | CDR2 | CDR3 | CDR1 | CDR2 | CDR3 |
| #132-4 | IgG1 | GFTFRNYD | ISPSGGSI | ARHGHTQFAY | QSLVHSDGNTY | RVS | LQSAHFPLT |
| #128-1 | IgG2b | GFTFSNYD | ITTSGGST | SRHRYNYH | QNINKY | NKN | FQHNSWPLT |
| #137-1 | IgG2b | GFTFSNYD | ITTSGGNT | TRGRYNSR | QNINKY | NTN | FQHDTWPLT |
| #148-1 | IgG2b | GFTFSKYD | ITTSGGST | ARHGRRLDY | QNINKY | NTN | FQHNSWPVT |
| #132-1 | IgG2b | GFTFSNFD | ITTRGGST | ARHGRYLAS | QNINRY | NSN | LQHDSWPLT |
| #118-1 | IgG2b | GFTFSTYD | ITFDGGRT | TTGRLTR | QNINRY | KAN | LQHDSWPLT |
| #122-1 | IgG2a | GFTFSNYD | ITTSGGST | ARHRNFAY | QNINKY | NTN | FQHNSWPLT |
| #147-1 | IgG2a | GFTFSNYD | ITTSGGNT | TRHRYLHL | QNIDKY | NTN | FQHNSWPLT |
| #144-3 | IgG2a | GFTFDDYG | ISWGGRSI | TRVGGGPYWSFDF | QDIGNY | GAT | LQHKQYPPT |
| #17-9 | IgG2a | GFTFSDYY | ISYDGGST | TTKLG | QSLLGTSGKTF | QVS | WQGTHFPDT |
| #126-1 | IgG2a | GYTFTDYY | INPNSGYT | TTGSFDY | QSLVYSDGKTY | QVS | VQARHFPFT |
| #121-1 | IgG2a | GYTFTSYY | INTRSGGT | ARSTFDY | QSLLDNDGNTY | LVS | MQANHAPLT |

**Table 10-1**

| Anti-A1 beta-casein antibody (II) derived from mouse | | | | | | | |
|---|---|---|---|---|---|---|---|
| No. | Subclass | VH | | | VL | | |
| | | CDR1 | CDR2 | CDR3 | CDR1 | CDR2 | CDR3 |
| m3-1-16 | IgG1 | GYTFTTYT | FNPSSGYT | AVYDGYFPFEY | QSLLNSRTRKNY | WAS | QQSYNLPWT |
| m19-2-1 | IgG1 | GYTFTSYT | INPSSGYT | AVYDGYFPFDY | QSLLNSRTRKNY | WAS | KQSYNLPWT |
| m22-4-11 | IgG1 | GYTFTSYT | INPSSGCS | SVYDDYFPFDY | QSLLNSRTRKNY | WAS | QHSYNLPWT |
| m4-1 | IgG1 | GSPLTSYT | IRPSSGYT | AVFDGYFPFDY | QSLLNSRTRKNY | WAS | KQSYNLPWT |
| m5-9 | IgG1 | GYTFSSYT | INPSSGYT | AGYSKDYPLDY | QSLLNSRTRNNY | WAS | KQSYNLPWT |
| m9-1 | IgG1 | GYTFTTST | INPSSGYT | AGYAKDYPMDY | QSLLNSRTRKNY | WAS | KQSYNLPWT |
| m100-1 | IgG1 | GYTFTDYS | INTETGEP | AGFHKRYPMDY | QSLFNSRTRKNY | WAS | KQSYNLPWT |
| m61-1 | IgG1 | GYTFTSYY | INPSSGGT | VYGNYRYPMDY | QSLLNRGNQKNY | WAS | QNDYSYPFT |
| m62-1- 2 | IgG1 | GYTFTSYY | INPSNGGT | VYGNYRYPMDY | QSLLNSGNRKNY | WAS | QNDYSYPFT |
| m128-1 | IgG1 | GYTFTTYW | INPGDGDT | VRPPHFYGSSLYAMDY | QSLLNSGKQKNY | WAS | QNDYSYPLT |
| m140-1-1 | IgG1 | GFDFSRYW | INPDSSTI | AREPHYYGNSYVRGY | QSLLNSRKQKNY | WAS | QNEYSYPLT |
| m139-1 | IgG1 | GFDFSRYW | INPDSSTI | AREPHYYGSSYVRGY | QSLLNSGKQKNY | WAS | QNDYSYPLT |
| m54-1 | IgG1 | GFDFSRYW | INPDSSTI | AREPHYYGSSYVRGY | QSLLNSGKQKNY | WAS | QNDYSYPLT |
| m22-1 | IgG1 | GYSFTSYW | IHPGNSDA | TRPPYYDYDEGDY | QSIVHSNGNTY | KVS | FQGSHVPFT |
| m25-1 | IgG1 | GYTFTNFG | INTYTGEP | ARRGVYNYDVGTWFPY | SSVNY | ATS | HQWSSHPH |
| m17-2-23 | IgG1 | GYTFTNFG | INTYTGEP | ARRGVYNYDVGTWFPY | SSVNY | ATS | HQWSSHPH |
| m136-1-2 | IgG1 | GYTFTNYG | INTYAGEP | ARWLRPDYYAMDY | KSVSTSGYSY | LVS | QHIRELTR |
| m93-1 | IgG1 | GYTFTNYG | INTYTGEP | ARRGFHYSNYAMAWFGY | KSVSTSGYSY | LVS | QHIRELTR |
| m19-4-22 | IgG1 | GYIFTNYG | INTYTGEP | ARRGYYGSYDPRTWFAY | KSVSTSGYSY | LVS | QHIRELTR |
| m38-1 | IgG1 | GYTFTNYG | INTYTGEP | TRRGFYGSHDPRTWFAY | KSVSTSGYSY | LVS | QHIRELTR |
| m24-32 | IgG2b | GYTFTNYG | INTYTGEP | ARRGVYNYDVGTWFAY | KSVSTSGYSY | LVS | QHIRELTR |
| m29-12 | IgG2b | GYTFTDHT | IFPNNGGT | ARSGGFAY | KSVSTSGYSY | LVS | QHIRELTR |
| m14-1-3 | IgG2b | GYSFTNYG | INTYTGEP | ARRGGSYRYDGAWFVY | QSLLDSDGKTY | LVS | WQGTHFIH |
| m126-1 | IgG2b | GYTFTNYG | INTYTGEP | ARNYYGISGDY | QSLVHSNGNTY | KVS | SQSTHVPYT |
| m67-2- 3 | IgG2b | GYTFTNYG | INTYTGEP | ARNYYGISGDY | QSLVHSNGNTY | KVS | SQSTHVPYT |
| m78-1 | IgG2b | GYTFTNYG | INTYTGEP | ARNYYGISGDY | QSLVHSNGNTY | KVS | SQSTHVPYT |
| m85-1 | IgG2b | GYTFTNYG | INTYTGEP | ARNYYGISGDY | QSLVHSNGNTY | KVS | SQSTHVPYT |
| m90-1 | IgG2b | GYTFTNYG | INTYTGEP | ARNYYGISGDY | QSLVHSNGNTY | KVS | SQSTHVPYT |
| m123-1 | IgG2b | GYTFTNYG | INTYTGEP | ARNYYGISGDY | QSLVHSNGNTY | KVS | SQSTHVPYT |
| m84-1 | IgG2b | GYTFTNYG | INTYTGEP | ARNYYGISGDY | QSLVHSNGNTY | KVS | SQSTQVPYT |
| m124-1 | IgG2b | GYTFTNYG | INTYTGEP | ARNYYGISGDY | QTLVHSNGNTY | KVS | SQSTLVPYT |
| m127-1 | IgG2b | GYTFTNYG | INTYTGEP | ARNYYGISGDY | QTLVHSNGNTY | KVS | SQSTLVPYT |
| m111-2 | IgG2b | GYTFTNYG | INTYTGEP | ARNYYGISGDY | QTLVHSNGNTY | KVS | SQSTLVPYT |

**Table 10-2**

| Anti-A1 beta-casein antibody (II) derived from mouse | | | | | | | |
|---|---|---|---|---|---|---|---|
| No. | Subclass | VH | | | VL | | |
| | | CDR1 | CDR2 | CDR3 | CDR1 | CDR2 | CDR3 |
| m77-1 | IgG2b | GYTFTNYG | INTYTGEP | ARNYYGISGDY | QTLVHSNGNTY | KVS | SQSTLVPYT |
| m88-1 | IgG2b | GYTFTNYG | INTYTGEP | ARNYYGISGDY | QTLVHSNGNTY | KVS | SQSTLVPYT |
| m98-1-1 | IgG2b | GYTFTNYG | INTYTGEP | ARNYYGISGDY | QTLVHSNGNTY | KVS | SQSTLVPYT |
| m109-1 | IgG2b | GYTFTNYG | INTYTGEP | ARNYYGISGDY | QTLVHSNGNTY | KVS | SQSTLVPYT |
| m117-1 | IgG2b | GYTFTNYG | INTYTGEP | ARNYYGISGDY | QTLVHSNGNTY | KVS | SQSTLVPYT |
| m112-1 | IgG2b | GYTFTNYG | LNTYTGEP | ARNYYGISGDY | QTLVHSNGNTY | KVS | SQSTLVPYT |
| m101-1 | IgG2b | GYTFTNYG | INTYTGQP | ARNYYGISGDY | QTLVHSNGNTY | KVS | SQSTLVPYT |
| m102-1 | IgG2b | GYTFTNYG | INTYTGQP | ARNYYGISGDY | QTLVHSNGNTY | KVS | SQSTLVPYT |
| m103-1 | IgG2b | GYTFTNYG | INTYTGQP | ARNYYGISGDY | QTLVHSNGNTY | KVS | SQSTLVPYT |
| m87-1 | IgG2b | GYTFTNYG | INTYTREP | ARNYYGISGDY | QTLLHSNGNTY | KVS | SQSTLVPYT |
| m70-1 | IgG2b | GYTFTNYG | INTYTGKP | ARNYYGISGDY | QSLVHSNGNTY | KVS | SQSTHVPYT |
| m71-1 | IgG2b | GYTFTNYG | INTYTGKP | ARNYYGISGDY | QSLVHSNGNTY | KVS | SQSTHVPYT |
| m81-1 | IgG2b | GYTFTNYG | INTYTGKP | ARNYYGISGDY | HGLVHSNGNTY | KVS | SQSTHVPYT |
| m120-1 | IgG2b | GYIFTNYG | INTYTGEP | ARNYYGISGDY | QSLVHSNGNTY | KVS | SQSTHVPYT |
| m130-1 | IgG2b | GYIFTNYG | INTYTGEP | ARNYYGISGDY | QSLVHSNGNTY | KVS | SQSTHVPYT |
| m110-1 | IgG2b | GYIFTNYG | INTYTGQP | ARNYYGISGDY | QGLVHSNGNTY | KVS | SQSTHIPYT |
| m108-1-1 | IgG2b | GYIFRNYG | INTYTGDP | ARNYYGISGDY | QGLVHSNGNTY | KVS | SQSTHIPYT |
| m99-1 | IgG2b | GYTFTNYG | INTYNGEP | ARNYYGISGDY | QSLVHSNGNTY | KVS | SQSTHVPYT |
| m69-1 | IgG2b | GFTFTNYG | INTYNGEP | ARNYYGISGDY | QSLLHSNGNTY | KVS | SQSTHVPYT |
| m113-1 | IgG2b | GFTFTNYG | INTYSGEP | ARNYYGISGDY | QSLLHSNGNTY | KVS | SQSTHVPYT |
| m82-1 | IgG2b | GFTFTNYG | INTYSGEP | ARNYYGISGDY | QSLLHSNGNTY | KVS | SQSTHVPYT |
| m115-1 | IgG2b | GYTFTDYG | INTYSGEP | ARNYYGISGDY | QSLVHSNGNTY | KVS | SQSTHVPYT |
| m107-1 | IgG2b | GYSFTNYG | INTYTGEP | ARNYYGISGDY | QSLVHSNGNTY | KVS | SQSTHVPYT |
| m121-1 | IgG2b | GYTFTNYG | INTYSGEP | GRNYYGISGDY | QSLVHSNGNTY | KVS | SQSTHVPYT |
| m68-1 | IgG2b | GYSLTKYG | INTYTGEP | GRNYYGISGDY | QSLVHSNGNTY | KVS | SQSTHLPYT |
| m51-23-1 | IgG3 | GYTFTNYG | INTYTGEP | ARNHYERAMDY | QSLVHSNGNTY | KVS | SQSAHVPWT |
| m141-1 | IgG3 | GYTFTNYG | INTYTGEP | ARNHYERAMDY | QSLVHSNGNTY | KVS | SQSAHVPWT |
| m143-2 | IgG3 | GYTFTNYG | INTYTGEP | ARNHYERAMDY | QSLVHSNGNTY | KVS | SQSAHVPWT |
| m144-1 | IgG3 | GYTFTNYG | INTYTGEP | ARNHYERAMDY | QSLVHSNGNTY | KVS | SQSAHVPWT |
| m151-1-1 | IgG3 | GYTFTNYG | INTYTGEP | ARNHYERAMDY | QSLVHSNGNTY | KVS | SQSAHVPWT |
| m145-1 | IgG3 | GYTFTNYG | INTYTGEP | ARNHYERAMDY | QSLVHSNGNTY | KVS | SQSTHVPWT |
| m53-1 | IgG3 | GYTFTNYG | INTYTGEP | ARNHYERGMDY | QSLVHSNGNTY | KVS | SQSTHVPWT |
| m142-1 | IgG3 | GYTFTNYG | INTYTGEP | ARNHYERSMDY | QSLVHSNGNTY | KVS | SQSTHVPWT |
| m147-1 | IgG3 | GYTFTNYG | INTYTGEP | ARNHYERAMDY | QSLVHINGNTY | KVS | SQSAHVPWT |

## Claims

1. A monoclonal antibody, comprising:
a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 303, and
a light chain variable region comprising a light chain complementarity determining region 1 comprising an amino acid sequence of SEQ ID NO: 304, a light chain complementarity determining region 2 comprising an amino acid sequence of SEQ ID NO: 305, and a light chain complementarity determining region 3 comprising an amino acid sequence of SEQ ID NO: 306,
wherein the monoclonal antibody binds to A1 beta-casein.

2. A monoclonal antibody, comprising:
a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 307, and
a light chain variable region comprising a light chain complementarity determining region 1 comprising an amino acid sequence of SEQ ID NO: 308, a light chain complementarity determining region 2 comprising an amino acid sequence of SEQ ID NO: 309, and a light chain complementarity determining region 3 comprising an amino acid sequence of SEQ ID NO: 310,
wherein the monoclonal antibody binds to A1 beta-casein.

3. A monoclonal antibody, comprising:
a heavy chain variable region comprising a heavy chain complementarity determining region 1 comprising an amino acid sequence of SEQ ID NO: 301, a heavy chain complementarity determining region 2 comprising an amino acid sequence of SEQ ID NO: 302, and a heavy chain complementarity determining region 3 comprising an amino acid sequence of SEQ ID NO: 311, and
a light chain variable region comprising a light chain complementarity determining region 1 comprising an amino acid sequence of SEQ ID NO: 312, a light chain complementarity determining region 2 comprising an amino acid sequence of SEQ ID NO: 313, and a light chain complementarity determining region 3 comprising an amino acid sequence of SEQ ID NO: 314,
wherein the monoclonal antibody binds to A1 beta-casein and A2 beta-casein.

4. The monoclonal antibody according to any one of claims 1 to 3, further comprising an Fc region.

5. A hybridoma comprising a nucleic acid coding the heavy chain variable region and the light chain variable region of the monoclonal antibody according to any one of claims 1 to 3.

6. A method for detecting A1 beta-casein in a milk sample, the method comprising the step of mixing the milk sample and the monoclonal antibody according to claim 1 or 2.

7. The method according to claim 6, further comprising the steps of:
selecting a detector antibody from the monoclonal antibody according to claim 1 or 2 and selecting a capture antibody from the monoclonal antibody according to any one of claims 1 to 3, wherein an epitope of the capture antibody is different from an epitope of the detector antibody,
immobilizing the capture antibody on a plate, and
mixing the detector antibody with a mixture of the capture antibody and the milk sample.
